Europäisches Patentamt

European Patent Office

Office européen des brevets

(11) Veröffentlichungsnummer: **0 595 241 A2**

# EUROPÄISCHE PATENTANMELDUNG

(21) Anmeldenummer: **93117282.9**

(22) Anmeldetag: **26.10.93**

(51) Int. Cl.5: **C12N 15/53**, A61K 39/395,
C12Q 1/32, C12P 21/08,
C12N 15/11, A61K 31/70

(30) Priorität: **28.10.92 DE 4236358**

(43) Veröffentlichungstag der Anmeldung:
**04.05.94 Patentblatt 94/18**

(84) Benannte Vertragsstaaten:
**AT BE CH DE DK ES FR GB GR IE IT LI LU MC
NL PT SE**

(71) Anmelder: **Boehringer Ingelheim International
GmbH**

**D-55216 Ingelheim(DE)**

(72) Erfinder: **Dworkin, Eva
Widerhoferplatz 4/43
A-1090 Wien(AT)**
Erfinder: **Loeber, Gerhard
Buchenweg 7
A-2380 Perchtoldsdorf(AT)**
Erfinder: **Schwendenwein, Renate
Braunspergengasse 12/2/16
A-1100 Wien(AT)**

(54) **Nachweis und Inhibierung von Malatenzym in Tumorzellen.**

(57) Hemmung von Wachstum und Vermehrung von Säugetier-Tumorzellen durch Inhibierung eines spezifisch in den Tumorzellen exprimierten Malatenzyms, insbesondere durch Inhibierung von zytosolischem $NADP^+$-abhängigem Malatenzym oder von mitochondrialem $NAD(P)^+$-abhängigem Malatenzym. Tumor-Diagnostika, die mit Malatenzym-Isoformen reagieren, insbesondere monoklonale Antikörper gegen zytosolisches $NADP^+$-abhängiges Malatenzym oder gegen mitochondriales $NAD(P)^+$-abhängiges Malatenzym. Arzneimittel für die Behandlung von humanen Tumorerkrankungen, enthaltend einen Inhibitor eines in Tumorzellen exprimierten Malatenzyms.

EP 0 595 241 A2

Die vorliegende Erfindung bezieht sich auf den Nachweis und die Inhibierung von Malatenzym in Tumorzellen.

Die Untersuchung des Stoffwechsels von Tumorzellen hat gezeigt, daß Glutamin und nicht Glukose die Hauptenergiequelle von Tumorzellen ist. Im Zellstoffwechsel spielt Pyruvat eine zentrale Rolle; es wird als Acetyl-CoA-Quelle für den Krebszyklus und für die Bildung von anabolischen Zuckerphosphaten benötigt. Die Umwandlung von Aminosäure-Kohlenstoff zu Pyruvat erfolgt unter Beteiligung von Malatenzym, das die oxidative Decarboxylierung von Malat zu Pyruvat katalysiert: Malat + $NAD(P)^+$ --> Pyruvat + $NAD(P)H^+$ + $H^+$. Es kommt sowohl in eukaryotischen als auch in prokaryotischen Zellen vor. Bisher wurden drei verschiedene Iso-Formen des Malatenzyms in Säugetiergeweben beschrieben: ein strikt zytoplasmatisches $NADP^+$-abhängiges Enzym (im folgenden als "Malatenzym 1" oder "ME 1" bezeichnet), eine $NADP^+$-abhängige mitochondriale Iso-Form (als "Malatenzym 2" oder "ME 2" bezeichnet) und ein mitochondriales Iso-Enzym, das sowohl $NAD^+$ als auch $NADP^+$ verwenden kann, im allgemeinen aber $NAD^+$ bevorzugt (Fraenkel, 1975). (Im folgenden wird diese Isoform als $NAD(P)^+$-abhängiges Malatenzym, vereinfacht auch als "Malatenzym 3" oder "ME 3" bezeichnet.)

Die Säugetier-Isoformen sind ca. 62-64 kDa groß (Moreadith und Lehninger, 1984b; Bagchi et al., 1987).

Eine native Größe von 240 kDA läßt für das aktive Enzym eine tetramere Struktur vermuten (Fraenkel, 1975; Moreadith und Lehninger, 1984b).

Die höchsten Aktivitäten des Malatenzyms 1 werden in der Leber und im Fettgewebe gefunden; dort steht diese Iso-Form in Verbindung mit der Bildung von zytosolischem NADPH für die de novo Fettsäure-Synthese. Dieses Iso-Enzym ist über die Nahrungszusammensetzung steuerbar und kann durch eine kohlenhydratreiche Diät oder durch Thyroidhormone induziert werden (Fraenkel, 1975; Dozin et al., 1985). $NADP^+$-abhängige mitochondriale Malatenzym-Aktivität (ME 2 Aktivität) kann in vielen Geweben (Gehirn, Herz- und Skelettmuskelgewebe, Nebenniere) nachgewiesen werden (Lin und Davis, 1974; Fraenkel, 1975; Nagel et al., 1980), wo sie möglicherweise mit der Produktion von NADPH für die reduktive Biosynthese verbunden ist (Simpson und Estabrook, 1969). Diese spezielle Iso-Form wurde jedoch bisher nicht näher charakterisiert.

Mitochondriale $NAD(P)^+$-abhängige Malatenzym-Aktivität kann in Geweben nachgewiesen werden, die hohe Zellteilungsraten aufweisen, z.B. in Thymus oder Milz oder in den Epithelzellen der Dünndarmschleimhaut (Sauer et al., 1979); weiters in Embryonen von Xenopus laevis während der ersten Zellteilungszyklen (Dworkin und Dworkin-Rastl, 1990). Die Aktivität dieser Iso-Form fehlt bzw. ist gering im Gehirn, im Muskelgewebe sowie in normalem und regenerierendem Lebergewebe der Ratte (Nagel et al., 1980), wurde jedoch in der Nebennierenrinde der Ratte (Sauer, 1973), im Skelettmuskel der Taube und des Menschen (Lin und Davis, 1974; Taroni et al., 1988) sowie im Herzmuskel verschiedener Spezies (Lin und Davis, 1974; Liguori et al., 1989) nachgewiesen. Sie wird in vielen von den bisher untersuchten Tumorzellinien aus Nagetieren exprimiert. $NAD(P)^+$-abhängige Malatenzym-Aktivität wurde in Mitochondrien von Ascitestumoren (Sauer und Dauchy, 1978), Hepatomzellen (Sauer et al., 1980) und verschiedenen anderen untersuchten Tumoren (Moreadith und Lehninger, 1984b) nachgewiesen. In der Morris-Hepatomserie ist die Expression von $NAD(P)^+$-abhängigem Malatenzym progressionsabhängig (Sauer et al., 1980).

Viele Tumorzellen in Kultur können als Hauptenergiequelle für den Metabolismus Glutamin ebenso wie Glukose verwenden (Reitzer et al., 1979), und viele maligne Zellinien haben nicht einmal einen absoluten Bedarf an Glukose per se (Wice et al., 1981). Die $NAD(P)^+$-gebundene mitochondriale Malatenzym-Aktivität dürfte mit der Umwandlung von Aminosäure-Kohlenstoff zu Pyruvat zusammenhängen (Moreadith und Lehninger, 1984a; Sauer et al., 1980).

In einer Arbeit von Nagel und Sauer, 1982, wird die teilweise Reinigung von mitochondrialem $NAD(P)^+$-abhängigem Malatenzym aus Hunde-Dünndarmschleimhaut beschrieben; Moreadith und Lehninger, 1984a, beschreiben die teilweise Reinigung (51 %) und Bestimmung der physikalischen Eigenschaften dieses Malatenzyms aus Maushepatom-Mitochondrien.

Humanes mitochondriales $NAD(P)^+$-abhängiges, in Tumorzellen exprimiertes Malatenzym (im folgenden als Tumormalatenzym 3 bezeichnet) in zur Homogenität gereinigter Form sowie seine Klonierung, die Expression des rekombinanten Enzyms sowie die Herstellung monoklonaler Antikörper wurde erstmals von Loeber et al., 1991, bzw. in der PCT-Anmeldung WO 92/04448 beschrieben.

Von Chang et al., 1990, wurden Versuche beschrieben, in denen das Tumorzellwachstum durch Dinukleotidanaloge gehemmt wurde. Es stellte sich jedoch heraus, daß die erzielte Inhibierung des Tumorzellwachstums unspezifisch ist und nicht durch Inhibierung des Malatenzyms verursacht wurde.

Der vorliegenden Erfindung lag die Aufgabe zugrunde, im Hinblick auf einen neuen Ansatz für eine effektive spezifische Tumordiagnose und -therapie ein Verfahren zum Nachweis und zur selektiven Inhibierung von in Tumorzellen überexprimiertem Malatenzym zu entwickeln.

Die Lösung der gestellten Aufgabe beruht auf der Überlegung, daß die zielgerichtete Inhibierung eines Malatenzyms, das in Tumorzellen gegenüber dem entsprechenden Normalgewebe überexprimiert wird, diese Tumorzellen in einen Wachstumsnachteil versetzt und somit ihr Wachstum und ihre Vermehrung inhibiert.

Um die Richtigkeit dieser Überlegung zu überprüfen, wurden zunächst humane Tumoren sowie verschiedene Tumorzellinien auf die Expression von Malatenzym-Isoformen untersucht. Ferner wurden im Zuge dieser Untersuchungen diejenigen Zellen innerhalb eines Tumorverbandes, die eine erhöhte Expression von Malatenzym aufweisen, genau charakterisiert.

Diese Charakterisierung stellt einerseits für sich einen diagnostischen Parameter dar, andererseits ist das Ergebnis dieser Charakterisierung die Voraussetzung dafür, durch spezifische Inhibierung von in Tumorzellen überexprimiertem Malatenzym gezielt das Wachstum und die Vermehrung von Tumorzellen zu hemmen.

Die vorliegende Erfindung betrifft somit ein Verfahren zur Inhibierung des Wachstums und der Vermehrung von Tumorzellen, bei dem man die Zellen auf Überexpression einer Malatenzym-Isoform gegenüber dem entsprechendem Normalgewebe untersucht und die Malatenzym-Isoform, die in den Zellen überexprimiert wird, inhibiert, indem man die Expression der Malatenzym-Isoform oder die Aktivität des Enzyms ganz oder teilweise inhibiert.

Die vorliegende Erfindung betrifft in einem weiteren Aspekt Mittel für den Nachweis der Expression eines Malatenzyms in Tumorzellen.

In einem bevorzugten Aspekt betrifft die vorliegende Erfindung Mittel zur Diagnose von Tumorerkrankungen von Säugetieren einschließlich des Menschen. Diese Mittel enthalten ein Reagens zum Nachweis eines Malatenzyms, vorzugsweise monoklonale Antikörper gegen eine spezifische Malatenzym-Isoform.

In einem weiteren Aspekt betrifft die vorliegende Erfindung Mittel zur Hemmung des Wachstums und der Vermehrung von Tumorzellen. Diese Mittel sind dadurch gekennzeichnet, daß sie die in den Tumorzellen überexprimierte Malatenzym-Isoform inhibieren.

In einem bevorzugten Aspekt betrifft die vorliegende Erfindung Mittel zur therapeutischen Behandlung von Tumorerkrankungen von Säugetieren, einschließlich des Menschen, die mit der Expression von Malatenzym 1 und/oder Malatenzym 3 einhergehen. Diese Mittel sind dadurch gekennzeichnet, daß sie einen Inhibitor für Malatenzym 1 und/oder Malatenzym 3 enthalten.

Im einzelnen wurde die erfindungsgemäß gestellt Aufgabe wie folgt gelöst.

Zunächst wurden humane Tumorzellinien auf ihren Gehalt an Malatenzym-Aktivität untersucht. Diese Werte wurden mit dem Gehalt an Malatenzym der entsprechenden normalen Zellen verglichen. Außerdem wurden humane Biopsien von Colonkarzinompatienten auf Malatenzym-Aktivität untersucht und die Aktivität im Tumorgewebe mit der entsprechenden Aktivität in normalem Gewebe verglichen. Ferner wurden monoklonale Antikörper und polyklonale Antiseren hergestellt, mit deren Hilfe Gewebsschnitte von Tumoren und normalem Gewebe auf das Vorhandensein von Malatenzym in situ untersucht wurden.

Humane Tumorzellinien, die aus verschiedenen Tumoren angelegt wurden, wurden, wie in den Beispielen beschrieben, geerntet und aufgearbeitet. Die erhaltener Rohlysate wurden entweder direkt über eine MonoQ-Ionenaustauschchromatographie von mit dem verwendeten Malatenzymassay interferierenden Enzymaktivitäten getrennt (z.B. Lactatdehydrogenase und Malatdehydrogenase) oder in zytosolische und mitochondriale Fraktionen aufgetrennt und daran anschließend durch Chromatographie gereinigt.

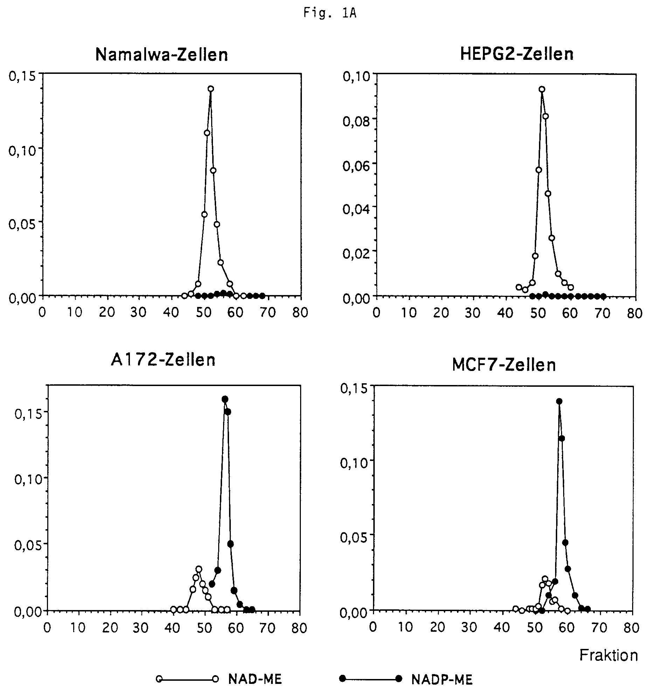

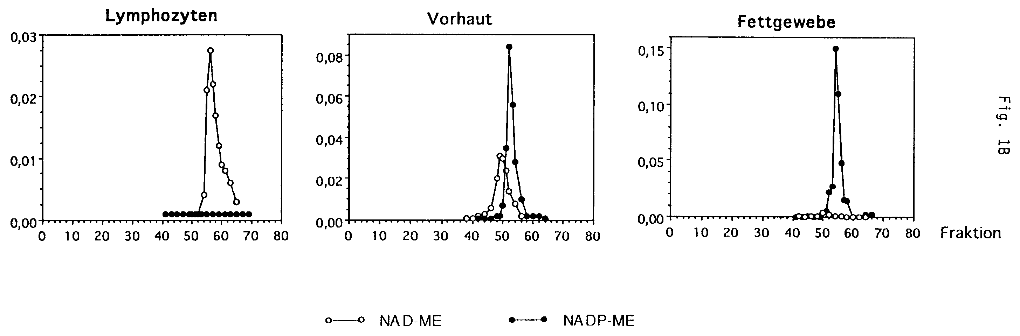

Fig. 1 zeigt das Auftrennungsprofil von Zell-Lysaten aus verschiedenen Tumorzellinien und normalen humanen Geweben durch einen 20 - 180 mM Salzgradienten (KCl). NAD(P)$^+$-abhängiges mitochondriales Malatenzym ME 3 eluiert bei einer KCl-Konzentration von 90 - 110 mM KCl, während NADP$^+$-abhängiges zytoplasmatisches Malatenzym ME 1 bei einer KCl-Konzentration von 100 - 130 mM KCl eluiert.

Die Ergebnisse aus der Untersuchung von Zellinien, die aus Leukämien angelegt wurden, sind in Tab. 1 zusammengefaßt. Alle diese Zellinien zeigen hohe Aktivitäten an NAD(P)$^+$-abhängigem mitochondrialem Tumor-Malatenzym ME 3. Die durchschnittlichen Aktivitäten liegen bei etwa 17 bis 30 mU Malatenzym pro mg Gesamtprotein. Vergleichbare normale humane Lymphozyten, die aus einer Standardreinigung von frischem humanen Vollblut isoliert wurden ("Buffy Coat Fraktion"), zeigen mit bis zu 4 mU Malatenzym pro mg Protein signifikant niedrigere Aktivitäten (Tab. 3). Humanes zytoplasmatisches NADP$^+$-abhängiges Malatenzym wurde weder in diesen Zellinien noch in normalen humanen Lymphozyten gefunden.

Ein deutlich anderes Bild zeigt sich in Zellinien, die aus soliden Tumoren angelegt wurden. Hierzu wurden Zellinien, die von Colon-, Lungen-, Brustkarzinomen sowie aus Hirntumoren angelegt wurden, untersucht (Tab 1 und 2). Während in Hepatomzellinien (Tab 1) und Colonkarzinomzellinien (Tab. 2) mit Ausnahme von CaCo-Zellen überwiegend hohe Aktivitäten an mitochondrialem NAD+-abhängigem Malatenzym ME 3 gefunden wurden, wurden in den Lungen- und Mammakarzinomzellinien sowie in der Glioblastomzellinie beide Isoformen zum Teil wesentlich stärker als im Ursprungsgewebe überexprimiert (Tab. 3).

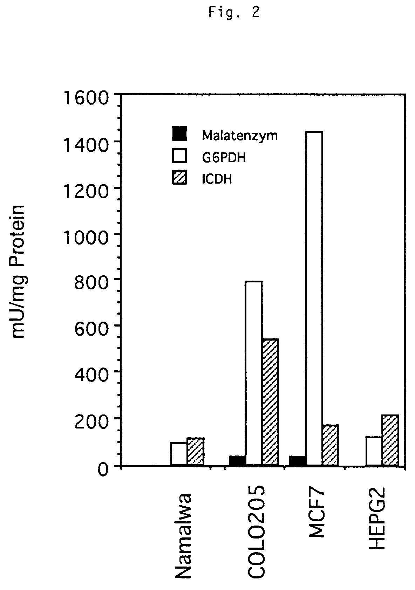

Um die subzelluläre Lokalisation der entsprechenden Malatenzyme genau zu charakterisieren, wurden die Zell-Lysate in mitochondriale und zytoplasmatische Fraktionen aufgetrennt. Dabei wurde festgestellt, daß in allen untersuchten Zellinien das NAD(P)$^+$-abhängige Malatenzym ME 3 ausschließlich mitochondrial lokalisiert ist, während NADP$^+$-abhängiges Malatenzym ME 1 nur in der zytoplasmatischen Fraktion zu finden ist (Tab. 4). Mitochondriales NADP$^+$-abhängiges Malatenzym ME 2, das in normalem Muskel, Herz und Hirngewebe zu finden ist, wird in keiner der untersuchten Zellinien nachweisbar exprimiert (Tab. 4). Die Rolle, die für zytoplasmatisches Malatenzym ME 1 bisher in der einschlägigen Literatur beschrieben wurde, ist die Bildung von reduzierenden Äquivalenten (NADP zu NADPH) für die Fettsäurebiosynthese in Leber und Adipozytenzellen, während die Bildung von Pyruvat aus Malat eher als Nebenprodukt betrachtet wird. Um die möglichen Quellen für die Erzeugung von reduzierenden Äquivalenten (NADPH) in den untersuchten Tumorzellen zu quantifizieren, wurden die Aktivitäten von zwei zytoplasmatischen NAPDH erzeugenden Enzymen, nämlich von Glucose-6-Phosphat-Dehydrogenase (G-6-PDH) und zytoplasmatischer Isocitratdehydrogenase (ICDH), mit der Aktivität von Malatenzym verglichen. Fig. 2 zeigt eine Zusammenfassung dieser Daten. Es ist aus dem Diagramm erkennbar, daß selbst in den Tumorzellen mit der höchsten Malatenzymaktivität die NADPH Produktion von G6PDH und ICDH diejenige von Malatenzym weit übersteigt. Dies bedeutet, daß in Tumorzellen die Signifikanz der Malatenzym-Reaktion nicht in der Verfügbarmachung von NADPH oder NADH für Fettsäuresynthese oder andere metabolische Prozesse liegt, sondern hauptsächlich in der Herstellung von Pyruvat.

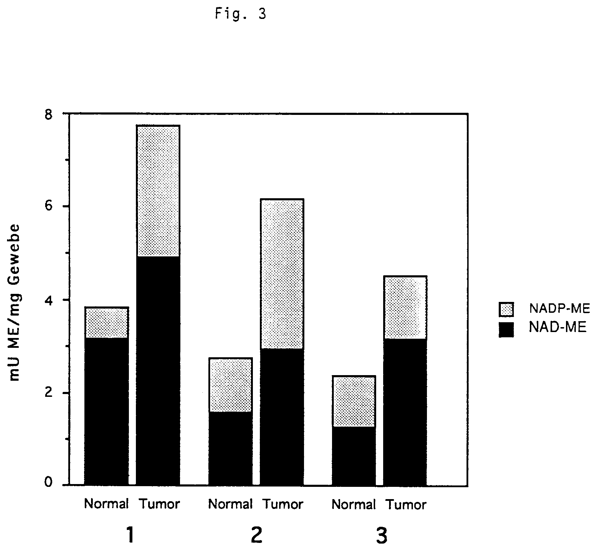

Ferner wurde die Aktivität der verschiedenen Malatenzym-Isoformen in humanen Colonkarzinomen untersucht. Das dafür notwendige Material wurde frisch nach Tumoroperationen erhalten. Dazu wurde das Tumorgewebe durch Pathologen fachmännisch von benachbartem normalen Gewebe getrennt. Die Gewebe wurden, wie in den Beispielen beschrieben, aufgearbeitet und die Aktivität von Malatenzym in den verschiedenen Tumoren mit der Malatenzym-Aktivität in benachbarter normaler Dickdarmschleimhaut derselben Patienten verglichen. Eine Zusammenfassung ist in Fig. 3 gezeigt (NAD-ME bedeutet Malatenzym 3, NADP-ME bedeutet Malatenzym 1). Bei diesem Vergleich zeigt sich, daß bei allen untersuchten Patienten eine Erhöhung der Aktivität des mitochondrialen NAD(P)$^+$-abhängigen Malatenzymes zu beobachten ist. Die Aktivität im Tumor ist im Vergleich zum benachbarten Normalgewebe etwa um den Faktor 2 bis 3 erhöht. Die Aktivität des zytosolischen Malatenzyms ME 1 ist bei zwei der drei untersuchten Patienten um das dreifache erhöht.

Die im Vergleich zu Tumorzellinien geringere Steigerung der Aktivität im Vergleich zum Normalgewebe ist damit zu erklären, daß ein Tumor nur zum Teil aus wachsenden Tumorzellen besteht und das isolierte Gewebe einen größeren Anteil an Bindegewebe und normalen Zellen als an Tumorzellen enthält. Daher ist ein Anstieg der spezifischen Malatenzym-Aktivität in den Tumorzellen eines Gesamttumors weniger leicht nachweisbar als in isolierten Tumorzellen in Kultur.

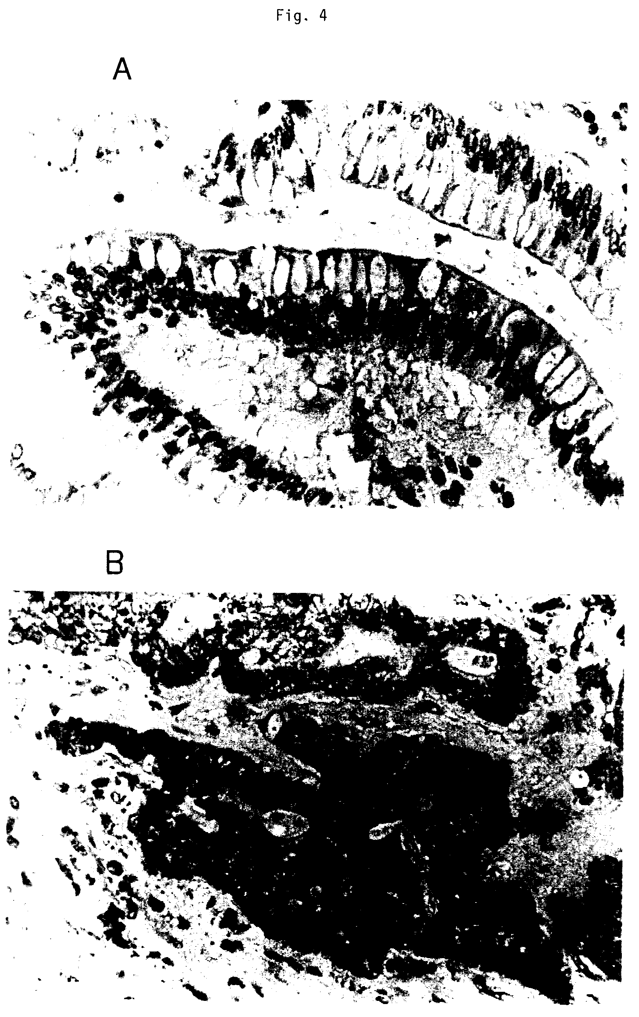

Gewebsschnitte von Colonkarzinomen wurden durch in situ Untersuchungen mit immunhistologischen Methoden auf die Korrelation von Malatenzym und Tumorzellwachstum untersucht (Fig.4).

Für diese Untersuchungen wurden Antiseren und monoklonale Antikörper gegen die verschiedenen Malatenzym-Isoformen hergestellt. Die verwendeten Antiseren reagieren überwiegend mit der jeweiligen Isoform des Malatenzyms.

Die überwiegende Zahl der untersuchten Colonkarzinombiopsien zeigte starke bis sehr starke Reaktivität mit dem M3-spezifischen Antiserum.

Die im Rahmen der vorliegenden Erfindung durchgeführten Versuche zeigten, daß beinahe alle untersuchten Tumorzellinien und Tumorgewebe eine starke Erhöhung der Aktivität mindestens eines Malatenzyms aufwiesen. Daraus wurde gefolgert, daß eine erhöhte Aktivität eines Malatenzyms wesentlich für das Wachstum der Tumorzellen ist. Diese Ergebnisse erhärteten die Richtigkeit der Überlegung, von der für die Lösung der gestellten Aufgabe ausgegangen wurde, nämlich daß die Inhibierung des in Tumorzellen exprimierten Malatenzyms zu einer Inhibierung des Wachstums und der Vermehrung dieser Tumorzellen führt.

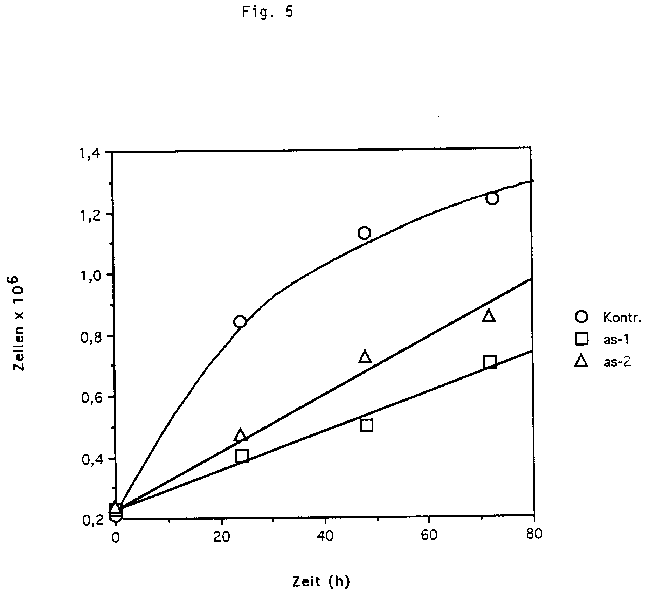

Die Richtigkeit der Hypothese wurde schließlich durch die im Rahmen der vorliegenden Erfindung durchgeführten Inhibierungsversuche bestätigt: es konnte gezeigt werden, daß eine Antisense-mRNA, die zu ME 1-Sequenzen komplementär ist und daher spezifisch die Expression von Malatenzym 1 verhindert, das Wachstum von Zellen der Tumorzellinie deutlich verlangsamt. Fig. 5 zeigt eine typische Wachstumskurve, bei der das Wachstum von zwei verschiedenen Klonen einer Lungenkarzinomzellinie (A549, ATCC No. CCL 185), transformiert mit einem Plasmid, von dem anti-ME 1-Antisense-RNA-Moleküle transkribiert werden, mit der parentalen normalen Tumorzellinie verglichen wird, die mit einem bis auf die Antisense-Sequenzen identischen Kontrollplasmid transformiert war. Das Wachstum der Zellinie, die mit Antisense-Oligonuklotiden transformiert war, ist um etwa den Faktor 3 langsamer als das der Vergleichszellinie.

Für die Diagnose und Therapie von Tumorerkrankungen wird zweckmäßig die folgende Strategie angewendet:

Mit Hilfe von monoklonalen Antikörpern gegen ein in Tumorzellen überexprimiertes Malatenzym, vorzugsweise solchen monoklonalen Antikörpern, die für eine Malatenzym-Isoform spezifisch sind, also nicht mit anderen Malatenzym-Isoformen kreuzreagieren, werden zunächst Tumorbiopsien auf Expression von Malatenzym-Isoformen untersucht. Derartige Untersuchungen umfassen vorteilhafterweise eine große Zahl von Biopsien aus Tumoren verschiedener Individuen, Gewebe und/oder Progressionsstadien. Das Ergebnis dieser Untersuchungen, insbesondere der Vergleich zur Expression der verschiedenen Malatenzym-Isoformen, erlaubt die Erstellung von Expressionsmustern von Malatenzym(en) in Abhängigkeit von bestimmten Tumorparametern. Die Ergebnisse derartiger Tumorbiopsie-Untersuchungen stellen die Grundlage für das Heranziehen von Malatenzym als Tumormarker und für die Verwendung von monoklonalen Anti-Malatenzym-Antikörpern als Tumordiagnostika dar. Eine für derartige Untersuchungen besonders geeignete Methode ist der Nachweis von Malatenzym(en) mittels Immunfluoreszenz, weil diese Methode den in situ Nachweis der Expression auf zellulärer Basis im Gewebeverband ermöglicht. Grundsätzlich sind zum Nachweis von Malatenzym sämtliche Methoden der immunologischen Bestimmung von Antigenen geeignet. Für die Routinediagnostik in der Klinik werden zweckmäßig die Tumorbiopsien aufgeschlossen und die Zellysate mittels herkömmlicher Immunoassays auf die Gegenwart von Malatenzym untersucht. Im Rahmen der vorliegenden Erfindung anwendbare Immunoassays beruhen auf Standardmethoden, die dem Fachmann auf dem einschlägigen Gebiet geläufig sind und von denen ein großes Angebot zur Verfügung steht. Diese Methoden basieren auf der Bildung eines Komplexes zwischen der zu bestimmenden antigenen Substanz und einem oder mehreren Antikörpern. Einer oder mehrere der Komplexpartner ist dabei markiert, sodaß das Antigen nachgewiesen und/oder quantitativ bestimmt werden kann.

Die Markierung kann z.B. in Form eines gekoppelten Enzyms, Radioisotops, Metallchelats, oder einer fluoreszierenden, chemilumineszierenden oder biolumineszierenden Substanz vorliegen.

Im Falle eines kompetitiven Immunoassays konkurriert das Antigen in der zu analysierenden Probe mit einer bekannten Menge von markiertem Antigen um die Bindung an die Antikörper-Bindungstellen. Die Menge von markiertem Antigen, gebunden an den Antikörper, ist daher verkehrt proportional zur Antigenmenge in der Probe.

Bei Tests, in denen markierte Antikörper verwendet werden, ist die Menge an markiertem, gebundenem Antikörper direkt proportional zur Menge an Antigen.

Assays, die auf der Bildung eines Antikörper/Antigen/Antikörper-Komplexes basieren, wobei zwei Antikörper eingesetzt werden, die einander bei der Bindung an das Antigen nicht behindern, werden als "Sandwich"-Immunoassays bezeichnet.

Bevorzugt werden die monoklonalen Antikörper gegen ME 1 oder ME 3 in einem Sandwich-Immunoassay, insbesondere in einem Sandwich-ELISA verwendet.

In einem weiteren Aspekt betrifft die vorliegende Erfindung Immunoassay-Kits, insbesondere Sandwich-Immunoassay-Kits, die anti-Malatenzym-Antikörper enthalten.

Eine bevorzugte Ausführungsform der vorliegenden Erfindung ist ein Sandwich-Immunoassay-Kit, vorzugsweise ein Sandwich-ELISA-Kit, in dem zwei verschiedene anti-Malatenzym-Antikörper, die mit derselben Isoform reagieren, den Beschichtungsantikörper und den markierten, vorzugsweise enzymgekoppelten, Antikörper darstellen.

Ein geeigneter Kit kann auch in getrennten Behältern Antikörper gegen verschiedene Malatenzym-Isoformen , z.B. gegen Malatenzym 1 und Malatenzym 3, enthalten

Geeignete Methoden für Assays sind einschlägigen Handbüchern zu entnehmen; s. z B. Harlow und Lane, 1988b.

Ein Beispiel für den diagnostischen Einsatz von Anti-Malatenzym-Antikörpern ist ein Kit, enthaltend monoklonale Antikörper gegen Tumor-Malatenzym 3, z.B. in fluoreszenzmarkierter Form. Dabei kann der Kit so aufgebaut sein, daß aufgrund der Spezifität des bzw. der Antikörper ein oder mehrere Malatenzym-Isoformen erfaßt werden können. Mit Hilfe eines derartigen Kits kann somit die Expression von NAD(P)$^+$-abhängigem Tumor-Malatenzym 3 und/oder seiner Isoformen, insbesondere Malatenzym 1, bestimmt und damit eine Tumordiagnose, z.B. eine Diagnose der Tumorprogression, gestellt werden.

Die Ergebnisse der Tumorbiopsie-Untersuchungen geben außerdem Aufschluß über spezifische Korrelationen der Expression von Malatenzym-Isoformen mit Parametern des Tumorwachstums.

Im Rahmen der vorliegenden Erfindung wurde das humane zytoplasmatische NADP$^+$-abhängige Malatenzym 1 kloniert und das rekombinante Malatenzym 1 in einem Baculovirus-Expressionssystem in Insektenzellen exprimiert.

Mit Hilfe des rekombinanten ME 1 ist es auf einfache Weise möglich, monoklonale Antikörper gegen humanes ME 1 herzustellen.

5

Die vorliegende Erfindung betrifft somit in einem weiteren Aspekt rekombinantes Malatenzym 1 sowie monoklonale Antikörper gegen ME 1.

Die Herstellung monoklonaler Antikörper ist dem Fachmann geläufig; geeignete Vorschriften können einschlägigen Handbüchern entnommen werden (z.B. Harlow und Lane; 1988a) bzw., falls erforderlich, entsprechend modifiziert werden.

Ein geeignetes Protokoll zur Herstellung von monoklonalen Anti-Malatenzym 1-Antikörpern besteht z.B. darin, Balb/c-Mäuse (im allgemeinen ist die Verwendung von drei Tieren zweckmäßig) dreimal hintereinander in Abständen von jeweils etwa 4 Wochen durch Injektion von rekombinantem gereinigtem Malatenzym 1 (z.B. ca. 100 $\mu$g einmal mit komplettem Freund'schem Adjuvans, das zweite Mal mit inkomplettem Freund'schen Adjuvans und das dritte Mal ohne Adjuvans) zu immunisieren. Nach ca. weiteren 10 Tagen wird den Mäusen Blut entnommen (nach diesem Zeitraum läßt sich im allgemeinen im Blutserum ein deutlicher Antikörper-Titer nachweisen). In diesen Seren wird der Antikörpertiter im Vergleich zu normalem Mausserum, das keine Reaktion zeigen sollte, bestimmt, z.B. in einem ELISA (Enzyme Linked Immunosorbent Assay). Dabei wird das rekombinante Malatenzym 1 zweckmäßigerweise direkt an die Oberfläche der Mikrotiterplatte gebunden und mit Antikörpern gegen Maus-Ig, gekoppelt an ein Enzym,

z.B. Meerrettich-Peroxidase, in Kontakt gebracht. Durch Reaktion mit dem entsprechenden Substrat kann, z.B. an Hand einer Farbreaktion, der Antikörper-Titer bestimmt werden. Von der Maus mit dem höchsten Antikörpertiter (im Falle nicht ausreichend hoher Titer muß gegebenenfalls nachimmunisiert werden) wird die Milz entnommen und die Milzzellen mit selektionierbaren (z.B. mittels HAT-Medium) Myelomzellen fusioniert. Geeignete Myelomzellen sind z.B. solche mit der Bezeichnung X-63-Ag8, X63-Ag.8.6.5.3., MPC-11, NS1-Ag4/1, MOPC-21 NS/1 oder SP 2/0; bevorzugt wird die Zellinie X63-Ag8.6.5.3. verwendet. Die Fusion wird durch Mischen der B-Lymphozyten und der Myelomzellen unter Zugabe eines Zellfusionsagens, wie Polyethylenglykol, Sendai-Virus, Calciumchlorid oder Lysolecithin durchgeführt. Vorzugsweise wird in Gegenwart von Polyethylenglykol, beispielsweise mit einem Molekulargewicht zwischen 1000 und 4000, fusioniert. (Durch die Verwendung von selektivem Kulturmedium wird gewährleistet, daß nur fusionierte Zellen, die sog. Hybridome, in Kultur überleben.) Die Hybridome werden kultiviert (z.B. in RPMI 1640 mit üblichen Zusätzen) und ihre Kulturüberstände, z.B. mittels oben beschriebenem ELISA auf die gewünschten Antikörper untersucht. Positive Polyklone werden - gegebenenfalls in mehreren Schritten - rekloniert, die resultierenden Monoklone erneut kultiviert und auf positive Reaktion mit Malatenzym 1 getestet. Positive Monoklone werden in größeren Mengen kultiviert. Die produzierten Antikörper werden aus Kulturüberständen oder Ascites gereinigt und charakterisiert. Aufgrund der abschnittsweise bestehenden Homologie des zytosolischen Malatenzyms mit dem mitochondrialen NAD(P)$^+$-abhängigen Malatenzym ist zu erwarten, daß sich unter den produzierten Antikörpern außer denen, die spezifisch mit Malatenzym 1 reagieren, d.h. keine Kreuzreaktivität mit den anderen Malatenzym-Isoformen zeigen, auch solche befinden, die außerdem an die mitochondriale NAD(P)$^+$-abhängige und/oder gegebenenfalls an die mitochondriale NADP$^+$-abhängige Isoform binden.

Die Nukleotidsequenzen der DNAs, die für die Malatenzym-Isoformen Malatenzym 1 bzw. Tumor-Malatenzym 3 kodieren, bieten die Voraussetzung für die Konstruktion von Antisense-Oligonukleotidmolekülen für die Inhibierung der Malatenzym-Expression und die Untersuchung der Auswirkungen dieser Inhibierung auf den Stoffwechsel und auf die Überlebens- und Proliferationsfähigkeit von Tumorzellen.

Der Fachmann auf dem einschlägigen Gebiet ist mit der Technologie inhibierender Oligonukleotide vertraut; es gibt zahlreiche Veröffentlichungen zu dieser Technologie, die in den letzten Jahren ständig weiterentwickelt wurde. Das Prinzip dieser Technik besteht darin, aufgrund einer bekannten Nukleotidsequenz zur mRNA komplementäre, gegebenenfalls modifizierte, Antisenseoligonukleotide, Antisense-mRNAs (s. z.B. die Übersichtsartikel von Green et al., 1986; Toulmé und Hélène, 1988) oder Ribozyme (s. z.B. EP-A2 321 201) zu konstruieren und damit eine Inhibierung der Malatenzym-Expression auf Translationsebene vorzunehmen. Bevorzugte Zielsequenzen für die Konstruktion von inhibierenden Nukleinsäuremolekülen sind Regionen im Bereich des Start-Codons sowie Regionen im 5'-nichttranslatierten Bereich, der deutliche Unterschiede zwischen den entsprechenden Sequenzen der verschiedenen Malatenzym-Isoformen aufweist.

Zur Entwicklung einer Tumortherapie auf Basis der Malatenzym-Inhibierung werden Zellinien, die Malatenzym exprimieren (von sich aus und/oder oder nach Transfektion mit einem Plasmid, enthaltend die für Malatenzym kodierende DNA-Sequenz) daraufhin untersucht, welchen Einfluß die Inhibierung von Malatenzym auf Wachstum und Vermehrung dieser Zellen hat. Dabei wird vorzugsweise so vorgegangen, daß die Zellen mit Plasmiden (co)transfiziert werden, die für inhibierende RNA-Moleküle kodierende DNA enthalten. Idealerweise sind diese Zellinien dann stabil mit dem entsprechenden Konstrukt transfiziert und exprimieren die inhibierenden mRNA Moleküle konstitutiv oder induzierbar. Mit Hilfe der in der Zelle gebildeten Transkripte wird die Expression von Malatenzym ganz oder teilweise verhindert. Geeignete Zellen sind z.B. von Humantumoren abgeleitete Zellinien. Nachdem festgestellt wurde, daß bzw. in welchem Ausmaß die Inhibierung von Malatenzym das Wachstum der Zellen beeinträchtigt, ist es vorteilhaft, in einem geeigneten Modellsystem immundefizienter Tiere (z.B. SCID-Mäuse oder Nacktmäuse) die Fähigkeit dieser Malatenzym reduzierten Zellinien zur Tumorbildung in vivo zu testen (im Vergleich zu den parentalen Zellinien). Dazu werden diese Tiere mit einer bestimmten Anzahl an normalen Tumorzellen und Vergleichstiere mit derselben Anzahl an Tumorzellen, in denen die Malatenzymaktivität inhibiert ist, inokuliert und die Häufigkeit der Tumorbildung und deren Wachstumsraten verglichen. Desweiteren kann in einem geeigneten transgenen Tiermodell, z.B. Mäusen, die Auswirkungen der Modulation der Malatenzymexpression im Gesamtorganismus von Säugetieren zu überprüft werden, z.B. anhand der gewebsspezifischen Expression von Anti-Malatenzym-Sequenzen oder der totalen Deletion von ME 3 in einem sogenannten "Knockout"-Mausmodell. Darunter wird im Rahmen der vorliegenden Erfindung eine transgene Maus verstanden, bei der ein Malatenzym-Gen durch homologe Rekombination gezielt ausgeschaltet wurde. (Knockout-Mäuse werden verwendet, um die biologische Funktion des betreffenden Genproduktes zu analysieren; ihre Herstellung und Verwendung ist in der Literatur beschrieben (Capecchi, 1989a, 1989b; Joyner, 1991). Im Hinblick auf eine Tumortherapie auf der Grundlage der Inhibierung von Malatenzym werden Knockout-Mäuse eingesetzt, um festzustellen, ob die Ausschaltung der betreffenden Isoform eine Schädigung des Organismus hervorruft bzw. welche Organe von einer gegebenenfalls eintretenden Schädigung betroffen sind.

Die Inhibierung von Malatenzym kann auf der Grundlage folgender Wirkprinzipien erfolgen:

1) Inhibierung durch niedermolekulare Substanzen, die die Bindungsstelle für das Substrat Malat blockieren:

Diese Substanzen haben die Eigenschaft, daß sie reversibel oder irreversibel mit hoher Affinität an die Malatbindungsstelle des Malatenzyms binden. Dadurch blockieren sie die Substratbindung und inhibieren als Folge davon die Enzymaktivität. Substanzen mit dieser Eigenschaft können prinzipiell mit Hilfe einer der folgenden Methoden identifiziert werden:
   a) aufgrund ihrer Ähnlichkeit mit Malat,
   b) durch Kristallisation des Malatenzyms und daraus abgeleitetes rationales Design oder
   c) durch ein "Random Screening"-Verfahren.
   Ein Random Screening-Verfahren, in welchem zweckmäßig Verdünnungen der zu untersuchenden Substanzen getestet werden, bzw. Tests zur Charakterisierung von potentiellen Malatenzym-Inhibitoren beruhen auf der Untersuchung der Inhibierung der Reaktion, die durch NAD(P)$^+$-abhängiges (ME 3) oder NADP$^+$ abhängiges (ME 1) Malatenzym katalysiert wird, durch die zu testenden Substanzen in vitro. Dabei werden zweckmäßig die jeweils zur Verfügung stehenden rekombinanten Malatenzym-Isoformen eingesetzt. An einen Inhibitor von ME 3 wird die Anforderung gestellt, daß er in niedriger Konzentration die Reaktion: Malat + NAD(P)$^+$ -> Pyruvat + NAD(P)H verhindert. Ob der potentielle Inhibitor diese Anforderung erfüllt, wird untersucht, indem z.B. in einem herkömmlichen Malatenzym-Assay sein etwaiger Einfluß auf die reaktionsbedingte Zunahme der Absorption bei 340 nm (Erhöhung der NAD(P)H Konzentration) bzw. daran gekoppelte Detektorsysteme charakterisiert wird. Vorzugsweise wird ein derartiger Assay, insbesondere bei einer großen Zahl von zu testenden Substanzen in einem Random Screening-Verfahren, in Mikrotiterplatten-gestützter, automatisierter Form, gesteuert von einem Robotics-System, eingesetzt.

Parallel zu einem derartigen Screening kann überprüft werden, ob und inwieweit die getesteten Substanzen auf andere Malatenzym-Isoformen wirken, um einen Inhibitor mit hoher Spezifität zu erhalten, sodaß eine gezielte Hemmung einer bestimmten Tumorisoform bewirkt wird. Substanzen, die in diesem in vitro Test die gewünschten Eigenschaften zeigen, werden hinsichtlich dieser Eigenschaften weiter in geeigneten Zellkultursystemen untersucht. Derartige Zellkulturen werden aus einem Tumor angelegt, im Hinblick auf die Therapie eines speziellen Tumors zweckmäßig aus demjenigen Tumor, der inhibiert werden soll. Die Herstellung solcher Zellkulturen wird nach Standardmethoden vorgenommen, die einschlägigen Fachbüchern entnehmbar sind, z.B. Freshney, 1986. Die Zellen werden auf ihre Eignung für ein zelluläres Testsystem überprüft, indem ihre Expression von Malatenzym 1 oder Malatenzym 3 durch Analyse der Zellysate überprüft wird. In diesen Versuchen wird, z. B. mittels Wachstumskurven, wie in Beispiel 8 dargestellt, festgestellt, inwieweit die Inhibitoren in die Tumorzellen aufgenommen werden und inwieweit sie in der Lage sind, das Wachstum der Tumorzellen zu beeinträchtigen. Im Hinblick auf einen Vergleich der inhibitorischen Wirkung verschiedener Substanzen empfiehlt sich die Standardisierung der Zellkultur- und Assaybedingungen.

In weiteren Schritten wird die Eignung der Substanzen für die Tumorinhibierung in vivo untersucht, vorzugsweise in Nacktmausmodellen. Dabei werden immundefiziente Mäuse mit humanen Tumoren angeimpft und anschließend mit den Inhibitor-Kandidaten behandelt. Geeignete Substanzen zeigen eine signifikante Verlangsamung des Tumorwachstums.

2. Inhibierung durch niedermolekulare Substanzen, die allosterische Funktionen des Malatenzymes beeinflussen.

Malatenzym 3 wird durch hohe ATP-Konzentrationen gehemmt und durch Dicarboxylsäuren wie Fumarat oder Succinat aktiviert. Daher ist es möglich, die Funktionen der Aktivatoren oder Inhibitoren durch niedermolekulare Substanzen gezielt zu beeinflussen. Geeignete Substanzen können durch Homologie zu den bekannten Aktivatoren oder Inhibitoren, durch "Random Screening" und durch sog. "rationales Drug Design" hergestellt werden. Der Test dieser Substanzen auf Brauchbarkeit als Inhibitor der jeweiligen Malatenzym-Isoform im Hinblick auf ihre Verwendung als Therapeutikum neoplastischer Erkrankungen entspricht der unter 1) angegebenen Vorgangsweise.

Das Prinzip der Strategie eines rationalen Drug-Designs wurde für die Inhibierung von Proteasen von Fauchère, 1986, beschrieben. Dieses Prinzip kann vom Durchschnittsfachmann auf das Design von Inhibitoren anderer Protein-Moleküle angewandt werden.

3. Inhibierung der Malatenzym-Expression durch Antisense-Oligonukleotide und Antisense-Oligonukleotid-Mimetika

Diese Art der Hemmung beruht auf der Inhibition der Expression von Malatenzym. Dazu werden geeignete Nukleotidsequenzen des zu inhibierenden Malatenzyms ausgewählt, die andere Malatenzym-Isoformen und verwandte Proteine nicht beeinflussen, also insbesondere Sequenzen, die möglichst geringe Homologie mit den anderen Malatenzym-Isoformen aufweisen, um gezielt die betreffende Tumorform des Malatenzyms auszuschalten. Die Antisense-Oligonukleotide können entweder auf RNA- oder DNA Basis hergestellt werden oder chemisch so modifiziert sein, daß eine leichtere Aufnahme in die Zielzelle ermöglicht wird oder daß eine Stabilisierung der Oligonukleotide erreicht wird. Die Oligonukleotide oder Oligonukleotid-Mimetika werden in geeigneten Zellkultursystemen untersucht (unter Oligonukleotid-Mimetika werden Substanzen verstanden, die sich hinsichtlich ihrer chemischen Struktur von Oligonukleotiden unterscheiden, jedoch dieselbe Wirkung haben, da sie spezifisch an eine bestimmte Targetsequenz auf der mRNA binden und dadurch die Translation inhibieren können). In diesen Versuchen wird festgestellt, inwieweit die Tumorzellen die inhibierenden Oligonukleotid-Moleküle aufnehmen und inwieweit diese in der Lage sind, das Wachstum der Tumorzellen zu beeinflussen. In weiteren Schritten wird die Brauchbarkeit der Oligonukleotide in vivo in geeigneten Nacktmausmodellen untersucht, bei der die immundefizienten Mäuse mit humanen Tumoren angeimpft werden und anschließend mit den Antisense-Oligonukleotiden behandelt werden. Geeignete Oligonukleotide zeigen eine signifikante Verlangsamung des Tumorwachstums.

4. Inhibierung der Malatenzym-Expression mittels Ribozymen

Dieser Mechanismus der Hemmung der Expression eines Proteins beruht auf der RNA-Inhibierung; es wurde erstmals von Haseloff und Gerlach, 1988, beschrieben. Die Inhibierung beruht auf der Fähigkeit von RNA-Molekülen, den sog. "Ribozymen", bestimmte RNA-Sequenzen zu erkennen, daran zu binden und sie zu spalten. Aufbauend auf bestimmten Strukturanforderungen für die Ribozym-katalysierte RNA-Spaltung können de novo Ribozyme konstruiert werden, die eine auf eine bestimmte Zielsequenz gerichtete in trans Endonukleaseaktivität aufweisen. Da solche Ribozyme, die wegen ihrer Struktur "hammer-head"-Ribozyme genannt werden, auf zahlreiche verschiedene Sequenzen wirken können, kann praktisch für jedes beliebige RNA-Substrat das entsprechende Ribozym "maßgeschneidert" werden. Dies macht Ribozyme zu interessanten, äußerst flexiblen Instrumenten für die Inhibierung spezifischer Gene und zu einer Alternative zu Antisense-Konstrukten, welche bereits einen potentiellen therapeutischen Nutzen gezeigt haben.

Das Ribozym-Modell umfaßt drei Strukturdomänen; aufbauend auf diesem Modell können Ribozyme konstruiert werden, die sich in vitro bereits sehr bald nach der Entdeckung dieses Mechanismus als für die effiziente und spezifische Spaltung von RNA-Sequenzen als geeignet erwiesen haben. (Haseloff und Gerlach, 1988). In der EP-A 387 775 sind Genkonstrukte beschrieben, die eine für ein Ribozym kodierende Sequenz und eine das Ribozym stabilisierende Sequenz enthalten.

5. Inhibierung der Malatenzym-Expression durch Beeinflussung der Gen-Kontrollsequenzen

Ein möglicher Zugang zur Inhibierung von Malatenzym kann auch über die Modulation seiner Expression auf der Grundlage der Wechselwirkung mit den regulatorischen Gensequenzen erfolgen; geeignete Vorversuche bestehen z.B. darin, zunächst die regulatorischen Sequenzen des zu inhibierenden Malatenzym-Gens mit einem geeigneten Reporter-Gen zu fusionieren, geeignete Zellen mit einem Plasmid, das diese Fusionssequenz enthält, zu transfizieren und den Einfluß von Substanzen auf die regulatorischen Sequenzen zu überprüfen, indem die Expression des Reporter-Gens bestimmt wird. Ein solches System kann ebenfalls automatisiert werden; Beispiele für derartige Screening-Systeme sind in der PCT-Anmeldung WO 91/01379 beschrieben.

Gegenstand der vorliegenden Erfindung ist somit in einem weiteren Aspekt die Verwendung eines Malatenzyms, vorzugsweise Malatenzym 1 und/oder Malatenzym 3, zum Screenen von Substanzen auf ihre Fähigkeit, die Aktivität des entsprechenden, in Tumorzellen exprimierten Malatenzyms zu inhibieren.

Gegenstand der Erfindung sind weiters DNA- oder RNA-Moleküle, die geeignet sind, die Expression eine in Tumorzellen exprimierten Malatenzyms zu inhibieren, indem sie die Translation der Malatenzym-mRNA verhindern.

Gegenstand der vorliegenden Erfindung sind in einem bevorzugten Aspekt Arzneimittel für die Behandlung von Tumorerkrankungen, die dadurch gekennzeichnet sind, daß sie einen Inhibitor für ein in Tumorzellen exprimiertes Malatenzym enthalten. Neben dem Inhibitor als wirksame Substanz enthält die pharmazeutische Zubereitung gegebenenfalls Zusatzstoffe. Jeder inerte pharmazeutisch annehmbare Träger kann verwendet werden, z.B. Kochsalzlösung oder phosphatgepufferte Kochsalzlösung oder jeder Träger, in dem die Inhibitoren geeignete Löslichkeitseigenschaften haben, um im Rahmen der vorliegenden Erfindung angewendet zu werden. Bezüglich Methoden zur Formulierung pharmazeutischer Zubereitungen wird auf Remington's Pharmaceutical Sciences, 1980, verwiesen. Die pharmazeutische Zubereitung liegt bevorzugt in Form einer Lösung vor, bevorzugte Anwendungsform ist die Injektion für die systemische Anwendung.

Figurenübersicht:

Fig. 1: Auftrennungsprofil von Lysaten verschiedener Tumorzellinien und von normalen humanen Geweben. Expression von Malatenzym 1 und Malatenzym 3. A: Tumorzellinien. B: Normalgewebe

Fig. 2: Expression von verschiedenen $NADP^+$-erzeugenden Enzymen in verschiedenen Tumorzellinien

Fig. 3: Expression von ME 1 und ME 3 in verschiedenen Colonkarzinomen und benachbarter normaler Colonmucosa

Fig. 4: Expression von ME 3 in einem humanen Colonkarzinom (gezeigt durch in situ Immunperoxidasefärbung)

Fig. 5: Wachstumskurve von Tumorzellinien, die anti-Malatenzym 1-Sequenzen exprimieren

Beispiel 1

Messung der Aktivität von NADP-abhängigem Malatenzym, Isocitratdehydrogenase, und Glucose-6-Phosphatdehydrogenase in Zellextrakten

Zellkulturen der humanen Tumorzellinien Namalwa (ATCC Nr. CRL 1432), Colo205 (ATCC Nr. CCL 222), MCF7 (ATCC Nr. HTB 22) und HepG2 (ATCC Nr. HB 8065) wurden in den jeweiligen Gewebekulturmedien (ATCC-Empfehlungen) gezüchtet. Pro Experiment wurde eine 175 $cm^2$ Gewebekulturflaschen verwendet, die Zellen befanden sich in der exponentiellen Wachstumsphase. Die Zellen wurden mit einem Gummiwischer geerntet und 3 x mit PBS gewaschen. Das Zellpellet wurde in kaltem 5 ml Extraktionspuffer (10 mM Tris-HCl pH 7.4; 3 mM $MgCl_2$, 1 mM EDTA, 20 mM KCl, 0.4 mM DTT, 0.5 mM Phenylmethylsulfonylflourid und 25 mM Glycerin aufsuspendiert und in einem Dounce-Homogenisator homogenisiert. Das Primärhomogenisat wurde mit 250 $\mu$l 10 NP40 versetzt und 4 x für je 20 sec mit Abkühlpausen von 1 min in einem Branson Ultraschallgenerator sonikiert. Anschließend wurde 30 min lang bei 40.000 rpm zentrifugiert. Der Überstand wurde durch ein 0.2 $\mu$m Filter filtriert. Nach Bestimmung des Proteingehaltes des Lysates wurden 100 $\mu$l des Lysates direkt in einem Assay für das entsprechende Enzym untersucht.

Assay auf Malatenzymaktivität: Das Reaktionsgemisch besteht aus: 50 mM Tris-HCl pH 7.4, 3 mM $MgCl_2$, 5 mM Malat, 0.15 mM $NADP^+$. Nach der Zugabe des Zell-Lysates wird in einem Beckmann DU64 Photometer die Extinktionszunahme bei einer Wellenlänge von 340 nm gemessen. 20 milliunits (mU) Enzymaktivität entsprechen einer Extinktionszunahme von 0.1 pro Minute, zur Auswertung wird die Extinktionszunahme auf eine Proteinmenge von 1 mg Totalprotein bezogen.

Assay auf Glucose-6-Phosphatdehydrogenaseaktivität: Das Reaktionsgemisch enthält: 20 mM Tris-HCl pH 7.4, 5 mM $MgCl_2$, 100 mM KCl, 2 mM Glucose-6-Phosphat und 0.15 mM $NADP^+$. Nach der Zugabe des Zell-Lysates wird in einem Beckmann DU64 Photometer die Extinktionszunahme bei einer Wellenlänge von 340 nm gemessen. 20 milliunits (mU) Enzymaktivität entsprechen einer Extinktionszunahme von 0.1 pro Minute, zur Auswertung wird die Extinktionszunahme auf eine Proteinmenge von 1 mg Totalprotein bezogen.

Assay auf Isocitratdehydrogenaseaktivität: Das Reaktionsgemisch enthält: 50 mM Tris-HCl pH 7.4, 3 mM $MgCl_2$, 1 mM Isocitrat, 0.15 mM $NADP^+$. Nach der Zugabe des Zell-Lysates wird in einem Beckmann DU64 Photometer die Extinktionszunahme bei einer Wellenlänge von 340 nm gemessen. 20 milliunits (mU) Enzymaktivität entsprechen einer Extinktionszunahme von 0.1 pro Minute, zur Auswertung wird die Extinktionszunahme auf eine Proteinmenge von 1 mg Totalprotein bezogen. Das Ergebnis dieser Versuche ist in Fig. 2 dargestellt.

Beispiel 2

Untersuchungen von Extrakten aus humanen Tumorzellinien

Zellkulturen der in den Tabellen 1 bis 3 angegebenen humanen Tumorzellinien wurden in den jeweiligen Gewebekulturmedien (ATCC-Empfehlungen) gezüchtet. Pro Experiment wurden drei 175 $cm^2$ Gewebekulturflaschen verwendet, die Zellen befanden sich in der exponentiellen Wachstumsphase.

Die Zellen wurden mit einem Gummiwischer geerntet und 3 x mit PBS gewaschen. Das Zellpellet wurde in kaltem 5 ml Extraktionspuffer (10 mM Tris-HCl pH 7.4; 3 mM $MgCl_2$, 1 mM EDTA, 20 mM KCl, 0.4 mM DTT, 0.5 mM Phenylmethylsulfonylflourid und 25 mM Glycerin aufsuspendiert und in einem Dounce-Homogenisator homogenisiert. Das Primärhomogenisat wurde 250 $\mu$l 10 NP40 versetzt und 4 x je 20 sec lang mit Abkühlpausen von 1 min in einem Branson Ultraschallgenerator sonikiert. Anschließend wurde für 30 min bei 40.000 rpm zentrifugiert. Der Überstand wurde durch ein 0.2 $\mu$m Filter filtriert.

Nach Bestimmung des Proteingehaltes des Lysates wurden 5 mg Protein direkt auf eine FPLC-MonoQ Säule (Pharmacia) aufgetragen. Die Malatenzymisoformen wurde durch einen 40 ml Gradienten (20 - 180 mM KCl) in 80 Fraktionen aufgetrennt. Malatenzym 1 eluiert bei Fraktion 58 - 60, Malatenzym 2 bei etwa 10 - 15 (breiter Peak) und Malatenzym 3 bei Fraktion 48 - 50. Die Aktivität der Peakfraktionen ($\Delta OD_{340}$ nm; 0.1 $\Delta OD/min$ = 20 mU) wurde aufsummiert und auf spezifische Aktivität (mU/mg Gesamtprotein) umgerechnet. Das Ergebnis dieser Versuche ist in den Tabellen 1 bis 3 bzw. in Fig. 1 dargestellt (NAD-ME bedeutet Malatenzym 3, NADP-ME bedeutet Malatenzym 1).

Beispiel 3

Fraktionierung von Zell-Lysaten in zytosolische und mitochondriale Fraktionen

Zellkulturen der in Tab. 4 angegebenen humanen Tumorzellinien wurden in den jeweiligen Gewebekulturmedien (ATCC-Empfehlungen) gezüchtet. Pro Experiment wurden drei 175 $cm^2$ Gewebekulturflaschen verwendet, die Zellen befanden sich in der exponentiellen Wachstumsphase.

Die Zellen wurden mit einem Gummiwischer geerntet und 3 x mit PBS gewaschen. Das Zellpellet wurde in kaltem 5 ml Extraktionspuffer (10 mM Tris-Hcl pH 7.4;, 1 mM EDTA, 219 mM Mannitol, 70 mM Saccharose) aufsuspendiert und in einem Dounce-Homogenisator homogenisiert. Das Primärhomogenisat wurde 10 min lang bei 1800 rpm zentrifugiert, um die Kerne abzutrennen. Der klare Überstand wurde erneut 20 min lang bei 10.000 rpm in einem Beckmann JS13-Rotor zentrifugiert. Der Überstand der Zentrifugation enthält die zytoplasmatische Fraktion. Das Pellet enthält die Mitochondrien und wird in 1.9 ml Extraktionspuffer (siehe oben) aufsuspendiert, mit 0.1 ml 10% NP-40 versetzt und 4 x 30 sec lang in einem Branson Ultraschallgenerator beschallt. Sowohl die zytoplasmatische als auch die mitochondriale Fraktion werden nochmals bei 40.000 rpm in einem Beckmann Ti50-Rotor zentrifugiert und die Überstände auf Malatenzymaktivität überprüft, wie in Beispiel 1 angegeben. Das Ergebnis der Malatenzym-Assays ist in Tab. 4 angeben.

Beispiel 4

Untersuchung von Tumorbiopsiematerial auf Malatenzymaktivität

Ca. 0.5 g gefrorenes Tumormaterial oder Normalgewebe wurden in einer Porzellanreibschale unter flüssigem Stickstoff zu einem feinen Pulver verrieben. Das gefrorene zerriebene Gewebe wurde in einem Dounce-Homogenisator in 5 ml Extraktionspuffer (10 mM Tris-Hcl pH 7.4; 3 mM $MgCl_2$, 1 mM EDTA, 20 mM KCl, 0.4 mM DTT, 0.5 mM Phenylmethylsulfonylflourid und 25 mM Glycerin) homogenisiert. Das Homogenat wird mit 250 $\mu$l 10 % NP40 versetzt und 4 x je 20 sec lang mit Abkühlpausen von 1 min in einem Branson Ultraschallgenerator sonikiert. Anschließend wurde für 30 min bei 40.000 rpm zentrifugiert.

Der Überstand wurde durch ein 0.2 $\mu$m Filter filtriert. Nach Bestimmung des Proteingehaltes des Lysates wurden 20 mg Protein direkt auf eine FPLC-MonoQ Säule (Pharmacia) aufgetragen. Die Malatenzymisoformen wurden durch einen 40 ml Gradienten (20 - 180 mM KCl) in 80 Fraktionen aufgetrennt. Malatenzym 1 eluiert bei Fraktion 58 - 60, Malatenzym 2 bei etwa 10 - 15 (breiter Peak) und Malatenzym 3 bei Fraktion 48 - 50.

Die Aktivität der Peakfraktionen ($\Delta OD_{340}$ nm; 0.1 $\Delta OD/min$ = 20 mU) wurde aufsummiert und auf spezifische Aktivität (mU/mg Gesamtprotein) umgerechnet.

Beispiel 5

Affinitätsreinigung der für ME 3 spezifischen Antikörper aus dem Kaninchen-Antiserum

Zur Herstellung der Affinitätssäule wurden 6.4 mg rekombinantes ME 3, hergestellt wie in der WO 92/04448 beschrieben, in 12 ml Puffer A plus 4 mM $NAD^+$ über Nacht gegen 2 x 800 ml Kupplungspuffer dialysiert, wobei nach 6 Stunden der Puffer einmal gewechselt wurde. Das dialysierte ME 3 wurde durch Zentrifugation durch Centricon 30-Filtereinheiten (5000 rpm, 20 min, 4°C) auf ein Volumen von 2 ml eingeengt. Dieses Material wurde mit 0.28 g CNBr-aktivierter Sepharose 4B, die durch Waschen mit 1 mM HCl bindungskompetent gemacht worden war, 2 h lang reagiert. Es ergab sich eine Bindung von 80 % des eingesetzten ME 3 an das Säulenmaterial. Weitere Bindung wurde durch Behandlung des Säulenmaterials mit 0.1 M Tris-HCl pH 8, über Nacht bei 4°C, verhindert. Hierauf wurde die Affinitätssäule gepackt und mit 5 Zyklen Bindungspuffer und Elutionspuffer (je 25 ml) gewaschen, wobei mit Bindungspuffer abgeschlossen wurde.

8 ml Kaninchenserum wurden mit 8 ml PBS versetzt und mit 16 ml gesättigtem $(NH_4)_2SO_4$ 30 min auf Eis gefällt. Das Präzipitat wurde mit 40 ml 50 % $(NH_4)_2SO_4$ gewaschen, in 2.7 ml PBS gelöst und gegen 4 x 1 l PBS + 0.02 % $NaN_3$ zwei Tage lang bei 4°C dialysiert. Hierauf wurde über Nacht bei 4°C gegen 1 l Bindungspuffer dialysiert. Das so erhaltene Material wurde auf die Affinitätssäule geladen, die Säule mit 40 ml Bindungspuffer gewaschen und die gebundenen Antikörper mit Elutionspuffer eluiert. Es wurden 1 ml-Fraktionen gesammelt und sofort durch Zugabe von 170 $\mu$l 1 M Tris-HCl, pH 9.5, neutralisiert. Die Fraktionen, die anti-ME 3-Antikörper enthielten (durch Messung der $O.D._{280}$ bestimmt) wurden gesammelt, durch Zugabe von 1 Volumen gesättigtem $(NH_4)_2SO_4$ 30 min auf Eis gefällt, mit 30 ml 50 % $(NH_4)_2SO_4$ gewaschen und in 2 ml PBS gelöst. Die Lösung wurde gegen 3 x 1 l PBS + 0.02 % $NaN_3$ dialysiert. Messung von $O.D._{280}$ ergab eine Gesamtmenge von 1.2 mg gereinigten anti-ME 3-Antikörpern, ein Wert, der sich aus der Beziehung von 1.4 $O.D._{280}$ = 1 mg/ml Immunoglobulin ergab.

| | |
|---|---|
| Kupplungspuffer: | 0.1 M $NaHCO_3$, pH 8.3<br>0.5 M NaCl |
| Bindungspuffer: | 0.1 M Tris-HCl, pH 8<br>0.5 M NaCl |
| Elutionspuffer: | 0.2 M Glycin, pH 2.3<br>0.5 M NaCl |
| PBS: | 0.130 M NaCl<br>7 mM $Na_2HPO_4$<br>3 mM Na $H_2$ $PO_4$ |

Beispiel 6

Routinefixiertes, in Paraffin eingebettetes und histologisch geschnittenes Biopsiematerial von Colonkarzinom-Patienten wurde vom Allgemeinen Krankenhaus der Stadt Wien zur Verfügung gestellt. Diese Schnitte wurden auf ihre Reaktivität mit anti-ME 3-Antikörpern getestet. Dazu wurde erst das Paraffin durch Behandlung mit Xylol (2 x 10 min) entfernt und die Schnitte in einer Alkoholreihe (je 5 min in 100 %, 95 %, 90 %, 85 %, 70 %, 50 %, 30 % Ethanol) hydriert und in $H_2O$ und PBS übergeführt. Hierauf wurden unspezifisch Protein bindende Stellen im Schnitt durch Inkubation in Blockierlösung (3 % Rinderserumalbumin in PBS) 80 min bei 4°C abgesättigt. Dann wurden die Schnitte kurz mit PBS gespült und 5 min mit 3 % $H_2O_2$ inkubiert, um endogene Peroxidaseaktivität zu zerstören. Nach Spülung mit PBS wurden die Schnitte über Nacht bei 4°C in einer feuchten Kammer mit 10 mg/ml affinitätsgereinigten anti-ME 3-Antikörpern inkubiert. Am nächsten Morgen wurden die Schnitte 2 x mit PBS gewaschen (30 min und 15 min), 1 h mit Blockierlösung inkubiert und hierauf mit dem sekundären Antikörper (Peroxidase-konjugierter Schwein-anti-Kaninchen Antikörper (Dakopatts) versetzt (1:100 verdünnt; Reaktion 4 h bei 4°C). Hierauf wurden die Schnitte 2 x mit PBS gewaschen (30 min und 15 min) und ME 3-Reaktivität durch Peroxidasereaktion mit Diaminobenzidin als Substrat sichtbar gemacht. Dazu wurden 6 mg Diaminobenzidin Tetrahydrochlorid in 10 ml 50 mM Tris-HCl, pH 7.6, gelöst und 0.1 ml einer 3%igen Lösung von $H_2O_2$ hinzugefügt. Die Schnitte wurden mit dieser Lösung 20 min inkubiert, mit Leitungswasser gewaschen, und 1 min mit Mayers Hämalaunlösung (Merck; 1:10 verdünnt) gegengefärbt.

Die Immunperoxidase-gefärbten Schnitte sind in Fig. 4 dargestellt: Colonkarzinomzellen zeigen signifikante Reaktivität in den von Tumorzellen gebildeten Tubuli, während in benachbarter normaler Colonmucosa keine signifikante Peroxidasereaktion zu sehen ist. Dies zeigt, daß eine signifikante Expression auf die Tumorbereiche beschränkt ist.

Tab. 5 zeigt eine Zusammenfassung der Ergebnisse aus den in situ Untersuchungen der Colonkarzinombiopsien (In der Tabelle wird die Intensität der Immunperoxidasereaktion in den Gewebeschnitten wie folgt angegeben: + + + = sehr stark, + + = stark, + = mittel ± = schwach). Normale Colonschleimhaut zeigte entweder eine schwache oder gar keine Reaktivität mit dem Antiserum. Über 50 % (= 28) der 54 untersuchten Colonkarzinombiopsien zeigten eine homogene Reaktion mit dem Antiserum, 27 davon hatten eine starke bis sehr starke Reaktion. Weitere 45 % zeigten eine inhomogen positive Reaktion, wobei eine Biopsie als inhomogen eingestuft wurde, in der weniger als 70-80 % der Tumorzellen Reaktivität zeigten. Lediglich 2 der insgesamt 54 Proben zeigten keine Reaktion. Gutartige Adenome des Dickdarms, die eine Vorstufe des malignen Wachstums darstellen und sich im späteren Verlauf zu einem Karzinom entwickeln können, zeigten eine zwischen normaler Colonschleimhaut und Colonkarzinomen liegende Reaktivität. Dies deutet darauf hin, daß das Malatenzym während der Tumorprogression induziert wird.

Beispiel 7

Isolierung eines vollständigen Klones von Malatenzym 1 und Expression von Malatenzym 1 in Insektenzellen

a) Isolierung eines vollständigen Klones von ME 1

Ein Sequenzvergleich der Proteinsequenzen von ME 1 aus Ratte und einem Malatenzym aus Mais-Chloroplasten zeigte komplette Konservierung der Sequenzen 241-Leu Ile Gln Phe Glu Asp Phe Ala Asn-249 und 354-Ala His Glu His Glu Glu-359. Es war daher zu erwarten, daß diese Sequenzen auch im humanen Malatenzym 1 vorhanden sind. Vollständig degenerierte Oligonukleotide, die den oben angegebenen Peptiden entsprechen, wurden hergestellt und in einer PCR Reaktion mit humaner HeLa-Zell-cDNA (Clontech No. HL 1022 b) eingesetzt, um ein Fragment spezifisch für humanes ME 1 zu erhalten. Folgende Bedingungen wurden für die PCR gewählt: 100 pM von jedem Primer, 0.2 mM dNTP, 10 μg humane HeLa Zell-cDNA, Taq-Reaktionspuffer nach den Empfehlungen des Herstellers der Taq-Polymerase (Promega) und 5 U Taq-Polymerase. Die PCR-Reaktion wurde unter folgenden Bedingungen durchgeführt: 45 sec 94°C, 60 sec 50°C, 3 min 72°C, 40 Zyklen total. Das erhaltene DNA Fragment wurde in einen puc18-Vektor subkloniert und in einem Standard Benton Davis Screen als Hybridisierungsprobe eingesetzt. Aus einer humanen Hippocampus cDNA Bibliothek (Stratagene 936205) wurde ein Klon der Länge von 2.058 bp isoliert, der die gesamte proteinkodierende Region enthielt. Die Nukleotidsequenz der cDNA und die davon abgeleitete Polypeptidsequenz sind im Sequenzprotokoll unter SEQ ID NO:3 dargestellt.

b) Expression von ME 1 in einem Baculovirus-Expressionssystem

Durch PCR Amplifikation wurde der gesamte kodierende Bereich von humanem ME 1 mit einer BamH1-Restriktionsstelle am 5'-Ende und einer Xba1-Restriktionsstelle am 3'-Ende versehen. Dazu wurden Oligonukleotide mit der Sequenz (SEQ ID NO:1) 5'-CTGAGGATCCATGGAGCCCGAAGCCCCCCGTCGCCGCCACA3' und (SEQ ID NO:2) 5'CTGATCTAGACTACTGGTCAACTTTGGTCTGTATT3' eingesetzt. Folgende Bedingungen wurden gewählt: 100 pM von jedem Primer, 0.2 mM dNTP, 100 ng klonierte humane cDNA von ME 1, Taq-Reaktionspuffer nach den Empfehlungen des Herstellers der Taq-Polymerase (Promega) und 5 U Taq-Polymerase. Die PCR-Reaktion wurde unter folgenden Bedingungen durchgeführt: 45 sec 94°C, 60 sec 60°C, 3 min 72°C, 20 Zyklen total. Das PCR Produkt wurde mit den Enzymen BamH1 und Xba1 geschnitten und in den entsprechend vorbereiteten Transfervektor pVL1393 (Pharmingen, San Diego) kloniert. Der Transfervektor wurde durch Standardmethoden (Maniatis) gereinigt und zusammen mit wt-Baculovirus-DNA in SF9-Zellen (ATCC CRL 1711) transfiziert. Rekombinante, humanes ME 1 exprimierende Klone wurden selektioniert und 2fach Plaque-gereinigt. Eine detaillierte Anleitung zur Klonierung in Baculoviren ist der einschlägigen Literatur zu entnehmen (King und Possee, 1991).

Das rekombinante humane ME 1 aus baculovirusinfizierten Insektenzellen zeigt dieselben enzymatischen Eigenschaften wie natürliches ME 1 aus humanen Zellen. Der $K_M$-Wert des Enzyms ist mit 0.7 mM (± 0.1 mM) identisch mit dem aus humanen Colo 205-Zellen (ATCC Nr. CCL 222) und unterscheidet sich deutlich vom endogenen $NADP^+$-abhängigen Malatenzym aus SF9-Zellen, das einen $K_M$-Wert von 0.2 mM (± 0.1 mM) von Malat hat.

Beispiel 8

Herstellung von Zellinien, die konstitutiv Antisense mRNAs zu ME 1 exprimieren und Messung des Wachstumsverhaltens dieser Zellinien

Beschreibung des Antisense-mRNA exprimierenden Konstruktes:

Das ME 1 Antisense Konstrukt besteht aus einer 1.2 kb langen Antisense Region der humanen ME 1 cDNA (bp 679-1861) unter der Kontrolle des Hühner $\beta$-Actin Promotors (Chang et al., 1984), und einer Polyadenylierungsstelle aus der Kaninchen $\beta$-Globin Genregion (Bgl2-Dra1: 152 bp-Fragment aus pSIL2.R$\alpha$; Hatakeyama et al., 1989). Dieses Konstrukt wurde zwischen die EcoRI-Schnittstelle (Position 89) und die Xho1-Schnittstelle der multiplen Klonierungsstelle des Vektors pMAMneo (Clontech 6104-1) kloniert. Durch diese Klonierung verliert der Vektor den induzierbaren MMTV-Promotor, aber enthält weiterhin neben einem bakteriellen Replikationsursprung auch ein Neomycin-Resistenzgen unter Kontrolle eines eukaryotischen Promotors.

Das Kontrollplasmid enthält nur den oben erwähnten Klonierungsvektor.

$10^7$ A549-Zellen (ATCC-CCL185) in einer 175 $cm^2$-Gewebekulturflaschewurden mit RPMI 1640 (inkl. 10 % hitzeinaktivierten fötalen Kälberserums, 100 Einheiten/ml Penicillin G und 50 Einheiten/ml Streptomycin) 1:10 verdünnt. Am darauffolgenden Tag wurden die Zellen trypsinisiert (2.5 g Trypsin, 2 g EDTA in einem Liter PBS, PBS = 0.2g KCl, 0.2g $KH_2PO_4$, 8.0g NaCl, 2.9g $Na_2HPO_4$ x 12 $H_2O$ pro Liter) und abzentrifugiert. Danach wurden die Zellen einmal mit PBS gewaschen und in PBS suspendiert (Dichte: 2 x $10^6$ Zellen pro 0.8 ml). 5 $\mu$g des oben beschriebenen Antisensekonstruktes bzw. des Kontrollplasmides wurden durch Elektroporation in 0.8 ml der Zellsuspension in einem Elektroporator (Typ Hoefer PG 200) transfiziert. Folgende Parameter wurden bei der Elektroporation verwendet: 1080 $\mu$Farad, 270 Volt, 1000 msec. Nach der Elektroporation wurden die Zellen 5 min bei Raumtemperatur (25°C) inkubiert und in Petrischalen (13 cm Durchmesser) ausgesät (Medium: RPMI 1640 wie oben). Am folgenden Tag wurde das Medium durch Selektionsmedium (RPMI 1640 + 10% FCS + 800 ng/ml G418 〈 Gibco〉) ersetzt.

Nach 14 Tagen wurden Neo-resistente Klone isoliert und expandiert. Zwei der mit ME 1 Antisense Plasmid transfizierten Klone und ein Kontrollklon (transfiziert mit dem Kontrollplasmid) wurden zur Erstellung von Wachstumskurven herangezogen.

Messung des Wachstums der Tumorzellen:

Je 0.2 x $10^6$ Zellen, die entweder mit dem Kontrollplasmid (Kontr.) oder mit einem Antisense-Konstrukt (as-1 und as-2) stabil transfiziert waren, wurden in 25 $cm^2$-Gewebekulturflaschen ausgesät (3 Flaschen pro Konstrukt). Nach 24, 48 und 72 Stunden wurde je eine Flasche geerntet, trypsinisiert und die suspendierten Zellen in einer Zählkammer gezählt. Die Anzahl der Zellen wurde gegen die Zeit aufgetragen, die Ergebnisse sind in Fig. 5 abgebildet. Die Wachstumskurven zeigen einen deutlichen Unterschied in der Wachstumsgeschwindigkeit zwischen der Kontroll-Zellinie und den beiden Zellinien, die mit den Antisensekonstrukten transformiert waren, die zu Beginn der Messungen um den Faktor 3 - 4 langsamer wuchsen.

Die Abflachung der Wachstumskurve bei der Kontrolle ist damit zu erklären, daß hier die Zellen am Ende der Messung annähernd konfluent waren.

Beispiel 9

Testung von ME3 spezifischen Inhibitoren auf Wachstumsinhibition von Tumorzellen

a) Identifikation von ME3 spezifischen Inhibitoren

Die durch "Random Screen" oder rationales Design aufgefundenen potentiellen Inhibitoren werden zunächst auf ihre Spezifität für ME3 getestet. Dazu werden die $IC_{50}$-Werte für des zu untersuchenden Inhibitors für verschiedene metabole Enzyme, deren Substrate oder Reaktionsprodukte Malat oder Pyruvat sind, getestet. Es werden insbesondere folgende Enzyme genau charakterisiert: Die drei verschiedenen Isoformen des humanen ME, humane Lactatdehydrogenase, humane Fumarase, humane cytoplasmatische sowie mitochondriale Malatdehydrogenase sowie humane Pyruvatdehydrogenase. Die Assaysysteme zur Aktivitätsbestimmung sowie zur $IC_{50}$-Bestimmung dieser Enzyme sind in der Literatur eingehend beschrieben (z.B. in S.P. Colowick, N.O. Kaplan (Herausgeber), Methods in Enzymology I-VII, 1955-1964). Ein ME3-spezifischer Inhibitor, der für die weitere Untersuchung in den nachfolgend beschriebenen Tests ausgewählt wird, inhibiert idealerweise ME3 im submikromolaren Bereich, während er die anderen Enzyme erst bei Konzentrationen von >500 $\mu$M beeinträchtigt.

b) Aufnahme der Inhibitoren in die Mitochondrien

Da ME3 ein mitochondriales Protein ist, wird vor Beginn von Zellkultur- oder in vivo Studien die Aufnahme des Inhibitors in die Mitochondrien überprüft. Zweckmäßigerweise werden dazu Tumorzell-Mitochondrien isoliert, die eine hohe ME3-Aktivität aufweisen. Mitochondrien werden z.B. aufgearbeitet, wie in R.W. Estabrook und M.E. Pullman, Methods in Enzymology X, 1967, beschrieben. Die isolierten Mitochondrien werden in einer Pufferlösung mit radioaktiv markiertem $^{14}$C-Malat versetzt. Isolierte Mitochondrien nehmen exogen zugesetztes Malat mit hoher Effizienz auf. Bei der durch ME3 katalysierten Reaktion wird radioaktives $CO_2$ aus dem Malat freigesetzt, das in NaOH aufgefangen und, z.B. in einem Szintillationszähler, quantitativ bestimmt wird. Durch parallelen Ansatz (mit und ohne Inhibitor) werden die Auswirkungen des Inhibitors auf die mitochondriale $CO_2$-Produktion aus Malat untersucht. Substanzen, die nicht in die Mitochondrien aufgenommen werden, können das in der mitochondrialen Matrix lokalisierte ME3 nicht inhibieren und somit auch die $CO_2$-Produktion nicht beeinflussen, während Substanzen, die die mitochondrialen Membranen durchqueren können, durch die Inhibierung des Malatenzyms eine Verringerung der Produktion von radioaktivem $CO_2$ aus Malat bewirken. Wenn die Konzentration einer exogen zugesetzten Substanz den 10fachen $IC_{50}$-Wert übersteigen muß, um einen signifikanten Einfluß auf die [14]$CO_2$-Produk-tion zu haben, wird sie ausgeschieden, weil ihre Aufnahme in die Mitochondrien offensichtlich unspezifisch ist.

c) Test der ME3-Inhibition in Zellkulturmodellen

i) Korrelation der Inhibition von ME3 mit der Zellproliferation

Als Testzellinien werden zweckmäßig Zellinien mit hoher ME3 Aktivität eingesetzt. Dazu bieten sich zum Beispiel REH, Namalwa oder Daudi-Zellen an. Von diesen Zellinien werden Wachstumskurven bei verschiedenen Inhibitorkonzentrationen gemessen. Die Wachstumskurven werden mit etablierten Assaysystem erstellt, die kommerziell erhältlich sind (z.B. MTS Proliferationsassay, Best. Nr. G 5421, Fa. Promega). Die Inhibitoren werden in Konzentrationen im Medium im Bereich zwischen 0,1 x $IC_{50}$ bis 10 x $IC_{50}$ eingesetzt. Von einem Inhibitor, der effizient in die Zellen aufgenommen und in die Mitochondrien transportiert wird, wird erwartet, daß er in dem getesteten Bereich einen signifikanten Effekt auf die Zellproliferation hat.

ii) Bestimmung der Korrelation zwischen Inhibitoreffekt und ME3 Gehalt einer Zellinie

In dieser Versuchsreihe wird der Effekt von ME3-spezifischen Inhibitoren auf das Wachstum von verschiedenen Tumorzellinien untersucht. Die Wachstumskurven für diese Versuchsreihen werden ermittelt, wie in i) beschrieben (MTS Assay). Dabei werden zweckmäßig parallel zwei Tumorzellinien eingesetzt, von denen die eine hohe Aktivität an ME3 aufweist (z.B. REH, HT29), während die andere Zellinie niedrige ME3 Aktivität aufweist (z.B. CaCo). Dabei werden Wachstumskurven für beide Tumorzellinien bei Inhibitorkonzentrationen gemessen, die in den in i) beschriebenen Tests zu einer signifikanten Reduktion der Tumorzellproliferation geführt haben. Von einem spezifischen Inhibitor ist zu erwarten, daß er Tumorzellinien, die auf eine hohe ME3 Aktivität angewiesen sind, bei diesen Konzentrationen signifikant inhibiert, während Zellinien mit einer geringen ME3 Aktivität (z.B. MCF-7, CaCo) normal bzw. fast normal proliferieren sollten.

Beispiel 10

Testung von Malatenzym-spezifischen Inhibitoren auf Wachstumsinhibition von Tumorzellen in vivo

Substanzen, die in den Screening Assays oder aufgrund von rationalem Design als potentielle Inhibitoren identifiziert wurden und die in den in Beispiel 9 beschriebenen zellulären Tests eine Hemmung der Proliferation von Tumorzellen gezeigt haben, werden anschließend in einem Tiermodell daraufhin untersucht, wie sie das Tumorwachstum unter in vivo Bedingungen beeinflussen können. Dazu wird als erstes die akute Toxizität in Mäusen getestet, um die ungefähre $LD_{50}$-Dosis zu bestimmen. Zusätzlich wird die Verträglichkeit einer längeren Verabreichung entsprechend den Schemen, die für derartige pharmakologische in vivo Versuche üblicherweise durchgeführt werden, untersucht.

Um die pharmakologische Aktivität zu bestimmen, werden diejenigen Tumorzellen, die auf die potentiellen Malatenzyminhibitoren in den in Beispiel 9 beschriebenen Versuchen am stärksten angesprochen haben, verwendet, um in vivo Testsysteme aufzubauen. Ihre subkutane Implantation in geeignete Mäusestämme resultiert in lokalem subkutanem Wachstum solider Tumore (subkutanes Modell). In loco Verabreichung von Tumorzellen, z.B. für Colonkarzinomzellen in der Darmwand, resultiert im Wachstum solider Tumore an der Stelle der Implantation (orthotopisches Modell). Um Maustumorzellen zu untersuchen, werden syngene immunkompetente Mäusestämme verwendet. Immundefiziente Mäuse, insbesondere Nacktmäusestämme, denen reife T-Lymphozyten fehlen, und SCID(Severe Combined Immunodeficiency)-Mäuse, denen sowohl T- als auch B-Lymphozyten fehlen, werden verwendet, um Xenotransplantate humaner Krebszellen zu untersuchen. Die Tiere, die solide Tumore gebildet haben, werden entweder intravenös, intraperitoneal oder per os mit den höchsten verträglichen Dosen des zu untersuchenden Malatenzyminhibitors behandelt. Die therapeutische Aktivität der Inhibitoren, die sich in einer Verringerung des Tumorwachstums in den subkutanen Modellen oder in einer Verlängerung der Überlebenszeit in den orthotopischen Modellen äußert, wird bestimmt. Anschließend werden Studien durchgeführt, in denen die dosisabhängige Wirkung derjenigen Malatenzyminhibitoren getestet wird, die in den ersten in vivo Untersuchungen eine Aktivität gezeigt haben.

# Tabelle 1:

Malatenzym-Aktivität in humanen Zellinien aus Leukämien
und aus Epstein Barr-Virus transformierten Zellen

|  | ME 1 | ME 3 |
|---|---|---|
| **EBV[1] transformierte Zellen** | | |
| 1788 (CCL 156) | 8.4 | 17.3 |
| **Leukämie-Zellinien** | | |
| REH (CRL 8286) | $\leq$0.3 | 17 |
| Namalwa (CRL 1432) | 0 | 23 |
| NC37 (CCL 214) | 0 | 29.6 |
| **Hepatomzellinien** | | |
| HepG2 (HB 8065) | 0 | 16 |

Die Aktivitäten sind in mU/mg Protein angegeben. [1]EBV = Epstein-Barr Virus. Die ATCC Bestellnummern der jeweiligen Zellinien sind in Klammern angegeben.

## Tabelle 2:

## Malatenzym-Aktivität aus Zellinien, die aus Karzinomen und Sarkomen abgeleitet wurden

|  | ME 1 | ME 3 |
|---|---|---|
| **Kolonkarzinomzellinien:** | | |
| Colo205 (CCL 222) | 6.2 | 10.9 |
| CaCo (HTB 37) | 11 | 3.5 |
| HT 29 (HTB 38) | 5.6 | 22.1 |
| DLD1 (CCL 221) | 15.9 | 15.8 |
| **Lungenkarzinomzellinien** | | |
| A549 (CCL 185) | 8.0 | 6.8 |
| **Brustkarzinomzellinien** | | |
| MCF7 (HTB 22) | 4.4 | 1.8 |
| **Glioblastomzellinie** | | |
| A172 (CRL 1620) | 4.2 | 6.5 |

Die Aktivitäten sind in mU/mg Gesamtprotein angegeben. Die ATCC Bestellnummern der jeweiligen Zellinien sind in Klammern angegeben.

Tabelle 3

| Malatenzym-Aktivität in humanen Normalgeweben | | |
|---|---|---|
| | ME 1 | ME 3 |
| Lymphozyten[1] | <0.2 | 2.5-4.0 |
| Erythrozyten | 0 | 0 |
| Brust (Fettgewebe) | 7 | $\leq 0.2$ |
| Brust (Muskel) | 0.7 | 0 |
| Placenta | 0.45 | 0.42 |
| Vorhaut | 2.7 | 1.8 |
| Colon-Mucosazellen | 0.5-1 | 1.5-3 |

Die Aktivitäten sind in mU/mg Gesamtprotein angegeben. [1] = Lymphozyten wurden durch eine "Buffy coat" Präparation aus humanem Vollblut isoliert.

## Tabelle 4:

### Subzelluläre Verteilung von Malatenzym in humanen Tumorzellen und normalem Gewebe

|  | Zytosol | Mitochondrien |
|---|---|---|
| Colo 205 - Zellen |  |  |
| ME 3 | 85 % | 15 % |
| ME 1 | 0 % | 100 % |
| RPMI 1788 Zellen |  |  |
| ME 3 | 92 % | 8 % |
| ME 1 | 1 % | 99 % |
| Normales Herzgewebe |  |  |
| ME 3 | 100 % | 0 % |
| ME 1 | 60 % | 40 % |
| Placenta |  |  |
| ME 3 | 100 % | 0 % |
| ME 1 | 0 % | 100 % |

Die Angaben zur Verteilung beziehen sich auf % der gesamten Aktivität an Malatenzym (% der Summe aus zytosolischer und mitochondrieller Malatenzymaktzivität). ATCC-Nummern der verwendeten Zellinien: Siehe Tab. 1 und 2.

## Tabelle 5:

## ME 3 Expression in humanen Colonkarzinomen und Adenomen

```
Colonkarzinom (n = 54)
        hom. pos.:      28 (22+++;  5++; 1+)
        inhom. pos.:    24 ( 6+++; 10++; 3+; 5±)
        neg.:            2


Colonadenom (n = 20)
        hom. pos.:       2 (2+++)
        inhom. pos.:    10 (1+++; 5++; 3+; 1±)
        neg.:            8


Normale Colonschleimhaut
        zwischen ± und neg.
```

Literatur:

Bagchi, S., Wise, L.S., Brown, M.L., Bregman, D., Sul, H.S. und Rubin, C.S., 1987, J. Biol. Chem. 262, 1558-1565.

Capecchi, M., 1989a, Science 244, 1288.

Capecchi, M., 1989b, Trends in Genetsic 3, March 1989, S.70.

Chang, K.S., Zimmer, W.E., Bergsma, B.J., Dodgson, J.B. und Schwartz, R.J., 1984, Mol. Cell. Biol. 4, 2498-2508.

Chang, G., Li, J., Chen, J., Meng, C., Lin, T. 1990, Int. J. Biochem. 22, 1259-1268.

Dozin, B., Magnusson, M.A. und Nikodem, V.M., 1985, Biochemistry 24, 5581-5586.

Dworkin, M.B. und Dworkin-Rastl, E., 1990, Dev. Biol. 138, 177-187.

Freshney, R.I., 1986, Animal Cell Culture - A Practical Approach., IRL Press, Oxford.

Fauchère, J.L., 1986, Advances in Drug Research 15, 29-69.

Fraenkel, R., 1975, Current Topics in Cellular Regulation 9, 157-181.

Green, P.J, Pines, O. und Inouye, M., 1986, Ann. Rev. Biochem. 55, 569-597

Hatakeyama, M. Tsudo, M., Minamoto, S., Kono, T., Doi, T., Miyata, T., Miyasaka, M. und Taniguchi, T., 1989, Science 244, 551-556.

Harlow, E. und Lane, D., 1988a, Antibodies, A Laboratory Manual, Cold Spring Harbor Laboratory, 139-237

Harlow, E. und Lane, D., 1988b, Antibodies, A Laboratory Manual, Cold Spring Harbor Laboratory, 359-420b

Haseloff, J. und Gerlach, W.L., 1988, Nature 334, 585-591.

Joyner, A., 1991, Bioassays 13(12), S.649.

King, L.A. und Possee, R.D., 1991, The baculovirus expression system. Chapman & Hall, London.

Liguori, M., Tessarolo, D., Ferro, M. und Giacanelli, M., 1989, Advances in Myochemistry 2, 203-204.

Lin, R.C. und Davis, E.J., 1974, J. Biol. Chem. 249, 3867-3875.

Loeber, G., Infante, A.A., Maurer-Fogy, I., Krystek E. und Dworkin, M.B., 1991, J. Biological Chemistry 266, 3016-3021

Maniatis, T., Fritsch, E.F. und Sambrook, J., 1982, Molecular Cloning A Laboratory Manual. Cold Spring Harbor Laboratory, 474

Moreadith, R.W. und Lehninger, A.L., 1984a, J. Biol. Chem. 259, 6215-6221.

Moreadith, R.W. und Lehninger, A.L., 1984b, J. Biol. Chem. 259, 6222-6227.

Nagel, W.O., Dauchy, R.T. und Sauer, L.A., 1980, J. Biol. Chem. 255, 3849-3854.

Nagel, W.O. und Sauer, L.A., 1982, J. Biol. Chem. 257, 12405-12411.

Reitzer, L.J., Wice, B.M. und Kennel, D., 1979, J. Biol. Chem. 254, 2669-2676.

Sauer, L., 1973, Biochem. Biophys. Res. Commun. 50, 524-531.

Sauer, L.A. und Dauchy, R.T., 1978, Cancer Res. 38, 1751-1756.

Sauer, L.A., Dauchy, R.T. und Nagel, W.O., 1979, Biochem. J. 184, 185-188.

Sauer, L.A., Dauchy, R.T., Nagel, W.O. und Morris, H.P., 1980, J. Biol. Chem. 255, 3844-3848.

Simpson, E.R. und Estabrook, R.W., 1969, Arch. of Biochemistry and Biophysics 129, 384-395.

Taroni, F., Gellera, C. und DiDonato, S., 1988, Biochem. Biophys. Acta 916, 446-454.

Toulmé, J.J. und Hélène, C., 1988, Gene 72, 51-58.

Wice, B.M., Reitzer, L.J. und Kennell, D., 1981, J. Biol. Chem. 256, 7812-7819.

Remington's Pharmaceutical Sciences, 1980, Mack Publ. Co., Easton, PA, Osol (ed.).

## Patentansprüche

1. Verfahren zur Inhibierung des Wachstums und der Vermehrung von Säugetier-Tumorzellen, dadurch gekennzeichnet, daß man die Tumorzellen darauf untersucht, ob sie Malatenzym und/oder welche Isoform von Malatenzym sie exprimieren und daß man das in den Zellen nachgewiesene Malatenzym spezifisch inhibiert, indem man seine Expression verhindert oder die Aktivität des Enzyms ganz oder teilweise inhibiert.

2. Verfahren nach Anspruch 1, dadurch gekennzeichnet, daß man humane Tumorzellen auf Expression von humanem zytosolischem $NADP^+$-abhängigem Malatenzym und/oder auf Expression von humanem mitochondrialem $NAD(P)^+$-abhängigem Malatenzym untersucht und die in den Zellen exprimierte Malatenzym-Isoform spezifisch inhibiert.

3. Verfahren zur Diagnose von Säugetier-Tumorerkrankungen, dadurch gekennzeichnet, daß man Material aus dem zu untersuchenden Individuum, insbesondere eine Tumorbiopsie oder eine daraus präparierte Probe, auf das Vorhandensein von zytosolischem $NADP^+$-abhängigem Malatenzym und/oder mitochon-

EP 0 595 241 A2

drialem NAD(P)$^+$-abhängigem Tumor-Malatenzym untersucht.

4. Mittel zur Durchführung des Verfahrens nach einem der Ansprüche 1 bis 3, dadurch gekennzeichnet, daß es spezifisch mit mitochondrialem NAD(P)$^+$-abhängigem Malatenzym reagiert.

5. Mittel nach Anspruch 4, dadurch gekennzeichnet, daß es einen monoklonalen Antikörper gegen mitochondriales NAD(P)$^+$-abhängiges Malatenzym enthält.

6. Mittel zur Durchführung des Verfahrens nach einem der Ansprüche 1 bis 3, dadurch gekennzeichnet, daß es spezifisch mit zytosolischem NADP$^+$-abhängigem Malatenzym reagiert.

7. Mittel nach Anspruch 6, dadurch gekennzeichnet, daß es einen monoklonalen Antikörper gegen zytosolisches NADP$^+$-abhängiges Malatenzym enthält.

8. Mittel zur Hemmung des Wachstums und der Vermehrung von Säugetier-Tumorzellen, dadurch gekennzeichnet, daß es ein Inhibitor eines in Tumorzellen exprimierten Malatenzyms ist oder diesen enthält.

9. Mittel nach Anspruch 8, dadurch gekennzeichnet, daß es ein Inhibitor für zytosolisches NADP$^+$-abhängiges Malatenzym ist oder diesen enthält.

10. Mittel nach Anspruch 8, dadurch gekennzeichnet, daß es ein Inhibitor für mitochondriales NAD(P)$^+$-abhängiges Malatenzym ist oder diesen enthält.

11. DNA, kodierend für humanes zytosolisches NADP$^+$-abhängiges Malatenzym.

12. DNA nach Anspruch 11 der Sequenz gemäß SEQ ID NO:3.

13. Rekombinantes humanes zytosolisches NADP$^+$-abhängiges Malatenzym.

14. Monoklonale Antikörper gegen humanes zytosolisches NADP$^+$-abhängiges Malatenzym.

15. Mittel nach Anspruch 9, dadurch gekennzeichnet, daß es zytosolisches NADP$^+$-abhängiges Malatenzym inhibiert, indem es seine Expression verhindert.

16. Mittel nach Anspruch 15, dadurch gekennzeichnet, daß es ein Antisenseoligonukleotid ist, das komplementär zu DNA-Sequenzen von zytosolischem NADP$^+$-abhängigem Malatenzym ist, die keine oder nur geringe Homologie zu DNA-Sequenzen von mitochondrialem NAD(P)$^+$-abhängigem Malatenzym aufweisen.

17. Mittel nach Anspruch 10, dadurch gekennzeichnet, daß es mitochondriales NAD(P)$^+$-abhängiges Malatenzym inhibiert, indem es seine Expression verhindert.

18. Mittel nach Anspruch 17, dadurch gekennzeichnet, daß es ein Antisenseoligonukleotid ist, das komplementär zu DNA-Sequenzen von mitochondrialem NAD(P)$^+$-abhängigem Malatenzym ist, die keine oder nur geringe Homologie zu DNA-Sequenzen von zytosolischem NADP$^+$-abhängigem Malatenzym aufweisen.

19. Verwendung von humanem zytosolischem NADP$^+$-abhängigem Malatenzym zum Untersuchen von Substanzen auf inhibierende Wirkung auf Wachstum und Vermehrung von humanen Tumorzellen.

20. Verwendung von humanem mitochondrialem NAD(P)$^+$-abhängigem Malatenzym zum Untersuchen von Substanzen auf inhibierende Wirkung auf Wachstum und Vermehrung von humanen Tumorzellen.

21. Arzneimittel, dadurch gekennzeichnet, daß es einen Inhibitor für humanes zytosolisches NADP$^+$-abhängiges Malatenzym enthält.

28

22. Arzneimittel, dadurch gekennzeichnet, daß es einen Inhibitor für mitochondriales NAD(P)$^+$-abhängiges Malatenzym enthält.

Fig. 1A

Fig. 1B

Fig. 2

Fig. 3

Fig. 4

A

B

Fig. 5

SEQUENZPROTOKOLL

(1) ALLGEMEINE INFORMATION:

    (i) ANMELDER:
        (A) NAME: Boehringer Ingelheim International GmbH
        (B) STRASSE: Binger Strasse 173
        (C) ORT: Ingelheim am Rhein
        (E) LAND: BRD
        (F) POSTLEITZAHL: 55216
        (G) TELEPHON: 06132/772282
        (H) TELEFAX: 06132/774377
        (I) TELEX: 4187910 bi d

    (ii) ANMELDETITEL: Nachweis und Inhibierung von Malatenzym in
        Tumorzellen

    (iii) ANZAHL DER SEQUENZEN: 4

    (iv) COMPUTER-LESBARE FORM:
        (A) DATENTRÄGER: Floppy disk
        (B) COMPUTER: IBM PC compatible
        (C) BETRIEBSSYSTEM: PC-DOS/MS-DOS
        (D) SOFTWARE: PatentIn Release #1.0, Version #1.25 (EPA)

(2) INFORMATION ZU SEQ ID NO: 1:

    (i) SEQUENZ CHARAKTERISTIKA:
        (A) LÄNGE: 41 Basenpaare
        (B) ART: Nukleinsäure
        (C) STRANGFORM: Einzel
        (D) TOPOLOGIE: linear

    (ii) ART DES MOLEKÜLS: cDNS

    (ix) MERKMALE:
        (A) NAME/SCHLÜSSEL: misc_feature
        (B) LAGE: 1..41

    (xi) SEQUENZBESCHREIBUNG: SEQ ID NO: 1:

CTGAGGATCC ATGGAGCCCG AAGCCCCCCG TCGCCGCCAC A                41

(2) INFORMATION ZU SEQ ID NO: 2:

    (i) SEQUENZ CHARAKTERISTIKA:
        (A) LÄNGE: 35 Basenpaare
        (B) ART: Nukleinsäure
        (C) STRANGFORM: Einzel
        (D) TOPOLOGIE: linear

21

(ii) ART DES MOLEKÜLS: cDNS

(ix) MERKMALE:
     (A) NAME/SCHLÜSSEL: misc_feature
     (B) LAGE: 1..35

(xi) SEQUENZBESCHREIBUNG: SEQ ID NO: 2:

CTGATCTAGA CTACTGGTCA ACTTTGGTCT GTATT          35

(2) INFORMATION ZU SEQ ID NO: 3:

    (i) SEQUENZ CHARAKTERISTIKA:
       (A) LÄNGE: 2058 Basenpaare
       (B) ART: Nukleinsäure
       (C) STRANGFORM: Doppel
       (D) TOPOLOGIE: linear

    (ii) ART DES MOLEKÜLS: cDNS

    (vi) URSPRÜNLICHE HERKUNFT:
       (A) ORGANISMUS: Homo sapiens
       (F) GEWEBETYP: Brain

    (ix) MERKMALE:
       (A) NAME/SCHLÜSSEL: CDS
       (B) LAGE: 1..1717

    (ix) MERKMALE:
       (A) NAME/SCHLÜSSEL: misc_feature
       (B) LAGE: 1..2058
       (D) SONSTIGE ANGABEN: /product= "humanes
           zytoplasmatisches NADP+-abhaengiges Malatenzym"

    (xi) SEQUENZBESCHREIBUNG: SEQ ID NO: 3:

```
ATG GAG CCC GAA GCC CCC CGT CGC CGC CAC ACC CAT CAG CGC GGC TAC   48
Met Glu Pro Glu Ala Pro Arg Arg Arg His Thr His Gln Arg Gly Tyr
 1               5               10                  15

CTG CTG ACA CGG AAC CCT CAC CTC AAC AAG GAC TTG GCC TTT ACC CTG   96
Leu Leu Thr Arg Asn Pro His Leu Asn Lys Asp Leu Ala Phe Thr Leu
             20              25                  30

GAA GAG AGA CAG CAA TTG AAC ATT CAT GGA TTG TTG CCA CCT TCC TTC   144
Glu Glu Arg Gln Gln Leu Asn Ile His Gly Leu Leu Pro Pro Ser Phe
         35              40                  45

AAC AGT CAG GAG ATC CAG GTT CTT AGA GTA GTA AAA AAT TTC GAG CAT   192
Asn Ser Gln Glu Ile Gln Val Leu Arg Val Val Lys Asn Phe Glu His
     50              55                  60
```

```
CTG AAC TCT GAC TTT GAC AGG TAT CTT CTC TTA ATG GAT CTC CAA GAT      240
Leu Asn Ser Asp Phe Asp Arg Tyr Leu Leu Leu Met Asp Leu Gln Asp
65                  70              75                  80

AGA AAT GAA AAA CTC TTT TAT AGA GTG CTG ACA TCT GAC ATT GAG AAA      288
Arg Asn Glu Lys Leu Phe Tyr Arg Val Leu Thr Ser Asp Ile Glu Lys
                85              90              95

TTC ATG CCT ATT GTT TAT ACT CCC ACT GTG GGT CTG GCT TGC CAA CAA      336
Phe Met Pro Ile Val Tyr Thr Pro Thr Val Gly Leu Ala Cys Gln Gln
                100             105             110

TAT AGT TTG GTG TTT CGG AAG CCA AGA GGT CTC TTT ATT ACT ATC CAC      384
Tyr Ser Leu Val Phe Arg Lys Pro Arg Gly Leu Phe Ile Thr Ile His
        115             120             125

GAT CGA GGG CAT ATT GCT TCA GTT CTC AAT GCA TGG CCA GAA GAT GTC      432
Asp Arg Gly His Ile Ala Ser Val Leu Asn Ala Trp Pro Glu Asp Val
        130             135             140

ATC AAG GCC ATT GTG GTG ACT GAT GGA GAG CGT ATT CTT GGC TTG GGA      480
Ile Lys Ala Ile Val Val Thr Asp Gly Glu Arg Ile Leu Gly Leu Gly
145             150             155             160

GAC CTT GGC TGT AAT GGA ATG GGC ATC CCT GTG GGT AAA TTG GCT CTA      528
Asp Leu Gly Cys Asn Gly Met Gly Ile Pro Val Gly Lys Leu Ala Leu
                165             170             175

TAT ACA GCT TGC GGA GGG ATG AAT CCT CAA GAA TGT CTG CCT GTC ATT      576
Tyr Thr Ala Cys Gly Gly Met Asn Pro Gln Glu Cys Leu Pro Val Ile
                180             185             190

CTG GAT GTG GGA ACC GAA AAT GAG GAG TTA CTT AAA GAT CCA CTC TAC      624
Leu Asp Val Gly Thr Glu Asn Glu Glu Leu Leu Lys Asp Pro Leu Tyr
        195             200             205

ATT GGA CTA CGG CAG AGA AGA GTA AGA GGT TCT GAA TAT GAT GAT TTT      672
Ile Gly Leu Arg Gln Arg Arg Val Arg Gly Ser Glu Tyr Asp Asp Phe
        210             215             220

TTG GAC GAA TTC ATG GAG GCA GTT TCT TCC AAG TAT GGC ATG AAT TGC      720
Leu Asp Glu Phe Met Glu Ala Val Ser Ser Lys Tyr Gly Met Asn Cys
225             230             235             240

CTT ATT CAG TTT GAA GAT TTT GCC AAT GTG AAT GCA TTT CGT CTC CTG      768
Leu Ile Gln Phe Glu Asp Phe Ala Asn Val Asn Ala Phe Arg Leu Leu
                245             250             255

AAC AAG TAT CGA AAC CAG TAT TGC ACA TTC AAT GAT GAT ATT CAA GGA      816
Asn Lys Tyr Arg Asn Gln Tyr Cys Thr Phe Asn Asp Asp Ile Gln Gly
                260             265             270

ACA GCA TCT GTT GCA GTT GCA GGT CTC CTT GCA GCT CTT CGA ATA ACC      864
Thr Ala Ser Val Ala Val Ala Gly Leu Leu Ala Ala Leu Arg Ile Thr
        275             280             285
```

```
AAG AAC AAA CTG TCT GAT CAA ACA ATA CTA TTC CAA GGA GCT GGA GAG      912
Lys Asn Lys Leu Ser Asp Gln Thr Ile Leu Phe Gln Gly Ala Gly Glu
    290                 295                 300

GCT GCC CTA GGG ATT GCA CAC CTG ATT GTG ATG GCC TTG GAA AAA GAA      960
Ala Ala Leu Gly Ile Ala His Leu Ile Val Met Ala Leu Glu Lys Glu
305                 310                 315                 320

GGT TTA CCA AAA GAG AAA GCC ATC AAA AAG ATA TGG CTG GTT GAT TCA     1008
Gly Leu Pro Lys Glu Lys Ala Ile Lys Lys Ile Trp Leu Val Asp Ser
                325                 330                 335

AAA GGA TTA ATA GTT AAG GGA CGT GCT TCC TTA ACA CAA GAG AAA GAG     1056
Lys Gly Leu Ile Val Lys Gly Arg Ala Ser Leu Thr Gln Glu Lys Glu
            340                 345                 350

AAG TTT GCC CAT GAA CAT GAA GAA ATG AAG AAC CTA GAA GCC ATT GTT     1104
Lys Phe Ala His Glu His Glu Glu Met Lys Asn Leu Glu Ala Ile Val
        355                 360                 365

CAA GAA ATA AAA CCA ACT GCC CTC ATA GGA GTT GCT GCA ATT GGT GGT     1152
Gln Glu Ile Lys Pro Thr Ala Leu Ile Gly Val Ala Ala Ile Gly Gly
        370                 375                 380

GCA TTC TCA GAA CAA ATT CTC AAA GAT ATG GCT GCC TTC AAT GAA CGG     1200
Ala Phe Ser Glu Gln Ile Leu Lys Asp Met Ala Ala Phe Asn Glu Arg
385                 390                 395                 400

CCT ATT ATT TTT GCT TTG AGT AAT CCA ACT AGC AAA GCA GAA TGT TCT     1248
Pro Ile Ile Phe Ala Leu Ser Asn Pro Thr Ser Lys Ala Glu Cys Ser
                405                 410                 415

GCA GAG CAG TGC TAC AAA ATA ACC AAG GGA CGT GCA ATT TTT GCC AGT     1296
Ala Glu Gln Cys Tyr Lys Ile Thr Lys Gly Arg Ala Ile Phe Ala Ser
            420                 425                 430

GGC AGT CCT TTT GAT CCA GTC ACT CTT CCA AAT GGA CAG ACC CTA TAT     1344
Gly Ser Pro Phe Asp Pro Val Thr Leu Pro Asn Gly Gln Thr Leu Tyr
        435                 440                 445

CCT GGC CAA GGC AAC AAT TCC TAC GTG TTC CCT GGA GTT GCT CTT GGT     1392
Pro Gly Gln Gly Asn Asn Ser Tyr Val Phe Pro Gly Val Ala Leu Gly
        450                 455                 460

GTT GTG GCG TGT GGA TTG AGG CAG ATC ACA GAT AAT ATT TTC CTC ACT     1440
Val Val Ala Cys Gly Leu Arg Gln Ile Thr Asp Asn Ile Phe Leu Thr
465                 470                 475                 480

ACT GCT GAG GTT ATA GCT CAG CAA GTG TCA GAT AAA CAC TTG GAA GAG     1488
Thr Ala Glu Val Ile Ala Gln Gln Val Ser Asp Lys His Leu Glu Glu
                485                 490                 495

GGT CGG CTT TAT CCT CCT TTG AAT ACC ATT AGA GAT GTT TCT CTG AAA     1536
Gly Arg Leu Tyr Pro Pro Leu Asn Thr Ile Arg Asp Val Ser Leu Lys
        500                 505                 510
```

```
ATT GCA GAA AAG ATT GTG AAA GAT GCA TAC CAA GAA AAG ACA GCC ACA    1584
Ile Ala Glu Lys Ile Val Lys Asp Ala Tyr Gln Glu Lys Thr Ala Thr
        515                 520                 525

GTT TAT CCT GAA CCG CAA AAC AAA GAA GCA TTT GTC CGC TCC CAG ATG    1632
Val Tyr Pro Glu Pro Gln Asn Lys Glu Ala Phe Val Arg Ser Gln Met
        530                 535                 540

TAT AGT ACT GAT TAT GAC CAG ATT CTA CCT GAT TGT TAT TCT TGG CCT    1680
Tyr Ser Thr Asp Tyr Asp Gln Ile Leu Pro Asp Cys Tyr Ser Trp Pro
545                 550                 555                 560

GAA GAG GTG CAG AAA ATA CAG ACC AAA GTT GAC CAG T AGGATAATAG       1727
Glu Glu Val Gln Lys Ile Gln Thr Lys Val Asp Gln
                565                 570

CAAACATTTC TAACTCTATT AATGAGGTCT TTAAACCTTT CATAATTTTT AAAGGTTGGA 1787

ATCTTTTATA ATGATTCATA AGACACTTAG ATTAAGATTT TACTTTAACA GTCTAAAAAT 1847

TGATAGAAGA ATATCGATAT AAATTGGGAT AAACATCACA TGAGACAATT TTGCTTCACT 1907

TTGCCTTCTG GTTATTTATG GTTTCTGTCT GAATTATTCT GCCTACGTTC TCTTTAAAAG 1967

CTGTTGTACG TACTACGGAG AAACTCATCA TTTTTATACA GGACACTAAT GGGAAGACCA 2027

AAATTACTAA TAAATTGAAA TAACCAACAT T                                 2058
```

(2) INFORMATION ZU SEQ ID NO: 4:

      (i) SEQUENZ CHARAKTERISTIKA:
        (A) LÄNGE: 572 Aminosäuren
        (B) ART: Aminosäure
        (D) TOPOLOGIE: linear

    (ii) ART DES MOLEKÜLS: Protein

    (xi) SEQUENZBESCHREIBUNG: SEQ ID NO: 4:

```
Met Glu Pro Glu Ala Pro Arg Arg Arg His Thr His Gln Arg Gly Tyr
 1               5                  10                  15

Leu Leu Thr Arg Asn Pro His Leu Asn Lys Asp Leu Ala Phe Thr Leu
            20                  25                  30

Glu Glu Arg Gln Gln Leu Asn Ile His Gly Leu Leu Pro Pro Ser Phe
            35                  40                  45

Asn Ser Gln Glu Ile Gln Val Leu Arg Val Val Lys Asn Phe Glu His
        50                  55                  60

Leu Asn Ser Asp Phe Asp Arg Tyr Leu Leu Leu Met Asp Leu Gln Asp
    65                  70                  75                  80
```

```
Arg Asn Glu Lys Leu Phe Tyr Arg Val Leu Thr Ser Asp Ile Glu Lys
                    85                  90                  95

Phe Met Pro Ile Val Tyr Thr Pro Thr Val Gly Leu Ala Cys Gln Gln
            100                 105                 110

Tyr Ser Leu Val Phe Arg Lys Pro Arg Gly Leu Phe Ile Thr Ile His
        115                 120                 125

Asp Arg Gly His Ile Ala Ser Val Leu Asn Ala Trp Pro Glu Asp Val
    130                 135                 140

Ile Lys Ala Ile Val Val Thr Asp Gly Glu Arg Ile Leu Gly Leu Gly
145                 150                 155                 160

Asp Leu Gly Cys Asn Gly Met Gly Ile Pro Val Gly Lys Leu Ala Leu
                165                 170                 175

Tyr Thr Ala Cys Gly Gly Met Asn Pro Gln Glu Cys Leu Pro Val Ile
                180                 185                 190

Leu Asp Val Gly Thr Glu Asn Glu Glu Leu Leu Lys Asp Pro Leu Tyr
        195                 200                 205

Ile Gly Leu Arg Gln Arg Arg Val Arg Gly Ser Glu Tyr Asp Asp Phe
        210                 215                 220

Leu Asp Glu Phe Met Glu Ala Val Ser Ser Lys Tyr Gly Met Asn Cys
225                 230                 235                 240

Leu Ile Gln Phe Glu Asp Phe Ala Asn Val Asn Ala Phe Arg Leu Leu
                245                 250                 255

Asn Lys Tyr Arg Asn Gln Tyr Cys Thr Phe Asn Asp Asp Ile Gln Gly
                260                 265                 270

Thr Ala Ser Val Ala Val Ala Gly Leu Leu Ala Ala Leu Arg Ile Thr
            275                 280                 285

Lys Asn Lys Leu Ser Asp Gln Thr Ile Leu Phe Gln Gly Ala Gly Glu
        290                 295                 300

Ala Ala Leu Gly Ile Ala His Leu Ile Val Met Ala Leu Glu Lys Glu
305                 310                 315                 320

Gly Leu Pro Lys Glu Lys Ala Ile Lys Lys Ile Trp Leu Val Asp Ser
                325                 330                 335

Lys Gly Leu Ile Val Lys Gly Arg Ala Ser Leu Thr Gln Glu Lys Glu
                340                 345                 350

Lys Phe Ala His Glu His Glu Glu Met Lys Asn Leu Glu Ala Ile Val
            355                 360                 365
```

26

```
Gln Glu Ile Lys Pro Thr Ala Leu Ile Gly Val Ala Ala Ile Gly Gly
        370             375             380

Ala Phe Ser Glu Gln Ile Leu Lys Asp Met Ala Ala Phe Asn Glu Arg
385             390             395             400

Pro Ile Ile Phe Ala Leu Ser Asn Pro Thr Ser Lys Ala Glu Cys Ser
            405             410             415

Ala Glu Gln Cys Tyr Lys Ile Thr Lys Gly Arg Ala Ile Phe Ala Ser
            420             425             430

Gly Ser Pro Phe Asp Pro Val Thr Leu Pro Asn Gly Gln Thr Leu Tyr
        435             440             445

Pro Gly Gln Gly Asn Asn Ser Tyr Val Phe Pro Gly Val Ala Leu Gly
        450             455             460

Val Val Ala Cys Gly Leu Arg Gln Ile Thr Asp Asn Ile Phe Leu Thr
465             470             475             480

Thr Ala Glu Val Ile Ala Gln Gln Val Ser Asp Lys His Leu Glu Glu
            485             490             495

Gly Arg Leu Tyr Pro Pro Leu Asn Thr Ile Arg Asp Val Ser Leu Lys
            500             505             510

Ile Ala Glu Lys Ile Val Lys Asp Ala Tyr Gln Glu Lys Thr Ala Thr
        515             520             525

Val Tyr Pro Glu Pro Gln Asn Lys Glu Ala Phe Val Arg Ser Gln Met
        530             535             540

Tyr Ser Thr Asp Tyr Asp Gln Ile Leu Pro Asp Cys Tyr Ser Trp Pro
545             550             555             560

Glu Glu Val Gln Lys Ile Gln Thr Lys Val Asp Gln
            565             570
```